# EUROPÄISCHE PATENTSCHRIFT

(11) **EP 1 961 371 B1**
(45) Veröffentlichungstag und Bekanntmachung des Hinweises auf die Patenterteilung: **08.03.2017**
(21) Anmeldenummer: 08001241.2
(22) Anmeldetag: 23.01.2008
(51) Int. Cl.: A61B 1/06

(54) **Beleuchtungsvorrichtung für eine Bilderfassungseinrichtung am distalen Ende eines Endoskops**
Lighting device for a picture capturing device at the distal end of an endoscope
Dispositif d'éclairage pour un dispositif de détection d'image au niveau de l'extrémité distale d'un endoscope

(30) Priorität: 31.01.2007 DE 102007005464; 30.03.2007 DE 102007015492
(43) Veröffentlichungstag der Anmeldung: 27.08.2008
(73) Patentinhaber: Fraunhofer-Gesellschaft zur Förderung der angewandten Forschung e.V., 80686 München (DE)
(72) Erfinder: Spinnler, Klaus, 91056 Erlangen (DE); Arnold, Cornelia, 07646 Renthendorf (DE); Kuleschow, Andreas, 90522 Oberasbach (DE); Rupp, Stephan, 91052 Erlangen (DE); Wittenberg, Thomas, 91054 Erlangen (DE)
(74) Vertreter: Schenk, Markus

(56) Entgegenhaltungen:
- WO-A1-01/73859
- WO-A1-95/15060
- WO-A1-02/059983
- JP-A- 2002 051 971
- US-A1- 2005 127 375
- US-A1- 2006 069 314
- US-A1- 2006 071 225
- US-A1- 2006 256 431

## Beschreibung

Die vorliegende Erfindung bezieht sich auf eine Beleuchtungsvorrichtung für eine Bilderfassungseinrichtung und ein Verfahren zum Betreiben einer Beleuchtungsvorrichtung und insbesondere auf eine Beleuchtungsvorrichtung für eine Bilderfassungseinrichtung am distalen Ende eines Endoskops.

Die Endoskopie ist eine wichtige, zerstörungsfreie Inspektionsmethode zur Untersuchung von im allgemeinen schwer zugänglichen, kleinen Hohlräumen in Medizin und Technik. Bei einem Endoskop wird ein distales Ende von einem proximalen Ende unterschieden. Das distale Ende bezeichnet das "Entfernte" Ende eines Endoskops, das in ein Objekt zur Beobachtung innerer Strukturen eingeführt wird, währenddessen das proximale Ende grundsätzlich außerhalb des untersuchten Hohlraums verbleibt. Dabei erfolgt durch das proximale Ende die Betrachtung des Bildes, z.B. mittels Blick in ein Okular oder durch Anschluss einer Kamera. Während in der Vergangenheit die endoskopische Inspektion manuell, durch den menschlichen Prüfer oder Arzt durchgeführt wurde, wird in jüngerer Zeit versucht, im Zuge der Entwicklung automatischer Sichtprüfsysteme auch vermehrt automatisierte, endoskopische Prüfsysteme zum Einsatz zu bringen.

Gerade im Bereich hydraulischer oder pneumatischer Bauelemente (z.B. Bremszylinder, Steuerelemente, etc.) mit funktionalen Bohrungsoberflächen bestehen hohe Qualitätsanforderungen, die oft eine vollständige Kontrolle der gesamten Produktion erfordern. Diese Prüfung bietet ein hohes Rationalisierungspotential, wenn entsprechende endoskopische Prüfautomaten zur Verfügung stehen.

Im wesentlichen sind zwei grundlegende Ansätze zur Beleuchtung von Hohlräumen bei deren endoskopischer Untersuchung bekannt.

Zum einen dienen Miniatur-Glühlampen, die z.B. am distalen Ende des Endoskops angebracht sind, zur Ausleuchtung der erfassten Szene oder Umgebung. Ein Nachteil ist die relative große Bauform, selbst von Miniatur-Glühbirnen, die den Einsatz bei sehr kleinen, dünnen Endoskopen unmöglich macht. Weiterhin problematisch bei dieser Technik ist der relativ hohe Anteil an Wärmestrahlung. Dies führt bei Glühlampen höherer Leistung schnell zu einem unakzeptabel großen Wärmeeintrag in das untersuchte Objekt.

Deshalb ist die heute überwiegend gebräuchliche Technik eine faseroptisch eingebrachte Kaltlichtbeleuchtung. Dabei befindet sich eine leistungsfähige Lichtquelle (z.B. eine Halogen-Glühlampe oder eine Lichtbogenlampe) außerhalb des Hohlraums. Das abgegebene Licht wird mittels Optiken (Spiegel, Kondensoren) gesammelt, durch faseroptische Lichtleiter zur Endoskopspitze übertragen und tritt dort am Ende der Lichtleitfasern aus. Eine Minimierung des übertragenen Infrarot-Anteils wird durch geeignete Infrarot-Sperrfilter erreicht. Das derart gefilterte Licht wird auch als "Kaltlicht" bezeichnet. Die Nachteile dieser Technik sind in der durch den Lichtaustritt am Faserbündel konstruktiv fest vorgegebenen Beleuchtungsanordnung, der verlustbehafteten Übertragung des Lichts sowie den erforderlichen, leistungsfähigen, teuren Kaltlichtquellen zu sehen.

In jüngster Zeit werden die externen Kaltlichtquellen auch durch externe LED-Lichtquellen mit Hochleistungs-LEDs ersetzt (LED = Light Emitting Diode oder Leuchtdiode oder ein Lichterzeugendes Halbleiterelement).

Bei der überwiegenden Anzahl der am Markt verfügbaren Endoskope ist die integrierte, faseroptische Beleuchtung in einer Dunkelfeld-Anordnung implementiert. Diese Art der Beleuchtung hat sich aufgrund der Vermeidung von Glanz- und Blendeffekten als besonders geeignet für den menschlichen Betrachter erwiesen.

Bei der Dunkelfeld-Anordnung wird ein lichtleitendes Faserbündel am Schaft des Endoskops angebracht, so dass der Lichtaustritt sich nahe neben dem oder auch koaxial um das distale Objektiv befindet. Dabei ist der Lichtaustritt am Faserbündel durch die Konstruktion des Gerätes fest vorgegeben, so dass sich außer der Gesamtintensität der Beleuchtung durch Regelung der externen Lichtquelle keine weiteren Beleuchtungsparameter beeinflussen lassen.

Bei der automatischen technischen Endoskopie erfolgt die Inspektion eines Hohlraumes (einer Bohrung) ohne menschliches Eingreifen durch ein Bildverarbeitungssystem, das zur automatischen Bildgewinnung ein Endoskop mit Videokamera und entsprechend geeigneter Beleuchtung motorisiert in den Hohlraum ein- und ausfährt. Die so gewonnenen Bilder werden automatisch mittels Bildverarbeitungs-Algorithmen ausgewertet. Somit wird eine Bewertung des geprüften Hohlraums (bzw. Bauteils) ermittelt, die ihrerseits zur Aussortierung fehlerhafter Teile genutzt werden kann.

Bei vielen Bauteilen hat sich zur automatisierten Untersuchung mittels Bildverarbeitungs-Algorithmen die üblicherweise von den Endoskop-Herstellern integrierte Dunkelfeldbeleuchtung als wenig geeignet und eine Hellfeld-Beleuchtung als vorteilhaft erwiesen. Unter Verwendung der Hellfeld-Beleuchtung sind bei der endoskopischen Bohrungsprüfung mehrere Anordnungen bekannt. Für DurchgangsBohrungen mit zwei Öffnungen erfolgt die Einbringung des Endoskops von der einen Seite der Bohrung, von der anderen Seite erfolgt die Einbringung der Beleuchtung z.B. in Form eines Lichtfingers. Als Lichtfinger wird eine stab- oder "finger"-förmige, starre oder flexible Vorrichtung bezeichnet, an deren Ende Licht austritt, z.B. mittels eines faseroptischen Lichtleiters. Die Bilderfassung erfolgt durch simultane Bewegung von Endoskop und Beleuchtung durch die Bohrung mit einem konstanten Abstand, so dass unter Berücksichtigung von Bohrungsdurchmesser, Bildwinkel der Optik, etc. eine konstante Hellfeld-Anordnung während der gesamten Bildaufnahme gewährleistet ist.

Dies ist bei Sackbohrungen, die nur eine Öffnung aufweisen, nicht möglich. Hier kann entweder die Lichtquelle an einem Träger vor der distalen Endoskopspitze befestigt und mit dem Endoskop in die Bohrung eingeführt werden. Dabei ist die Beleuchtung breit abstrahlend und auf die Optik gerichtet, so dass wiederum unter Berücksichtigung der geometrischen Randbedingungen eine Hellfeld-Beleuchtung erreicht wird. Ein anderer Ansatz arbeitet mit einem "Rundum-Retroblick". Dabei wird das Sichtfeld über einen geeigneten Spiegel vor dem Endoskop oder eine sog. Greguss-Linse rundum, umfänglich über 360 Grad seitlich oder nach rückwärts umgelenkt. Durch eine Beleuchtung, die rundum am Schaft des Endoskops, leicht zurückversetzt, angebracht ist, kann wiederum eine Hellfeld-Anordnung erreicht werden. Beiden Ansätzen gemeinsam ist der Nachteil, dass der Boden, bzw. der letzte Abschnitt der Bohrungswandung der Sackbohrung nicht erfasst werden kann.

Bezüglich den bisher eingesetzten Endoskopbeleuchtungen sollte aber beachtet werden, dass die heute am Markt verfügbaren Endoskope im Wesentlichen ausnahmslos Beleuchtungsvorrichtungen aufweisen, deren Funktionsweise für die Betrachtung des endoskopischen Abbildes durch den Menschen optimiert sind. Da in automatisierten, endoskopischen Prüfsystemen die gewonnenen Bilder nicht mehr vom Auge erfasst und vom Menschen bewertet werden, sondern von Kameras und bildverarbeitenden Mustererkennungs-Algorithmen in Computern erfasst und bewertet werden, bedarf es anderer, neuer Endoskop-Beleuchtungen, um Bilder in hoher Qualität für das maschinelle Erfassen (Maschinen-Sehen) zu gewinnen. Es besteht somit insbesondere ein Bedarf nach Beleuchtungsvorrichtungen für Endoskope, die die optimierte Bildgewinnung bei Sichtprüf-Automaten ermöglichen. Dadurch soll es möglich werden, eine große Zahl von schwierigen Prüfaufgaben an Bauteilen mit inneren Oberflächen mittels automatischer, endoskopischer Sichtprüfsysteme wirtschaftlich zu lösen, die bislang nicht oder nur mit hohem, personellen Aufwand und den damit verbundenen, hohen Kosten durchführbar waren.

Die WO95/15060A1 bezieht sich auf ein Einzelsensorvideobilderzeugungssystem und ein Verfahren unter Verwendung einer sequentiellen Farbobjektbeleuchtung.

Die US2006/0069314A1 bezieht sich auf eine Solid-State-Beleuchtung für ein Endoskop, wobei insbesondere unterschiedliche Konfigurationen für die Anordnung der Beleuchtung am distalen Ende eines Endoskops angegeben sind.

Die WO01/73859A1 bezieht sich auf eine lichtemittierende Diode mit gesteigerter Ausgabeleistung und auf ein Verfahren zum Herstellen desselben.

Ausgehend von diesem Stand der Technik liegt der vorliegenden Erfindung die Aufgabe zugrunde, eine Beleuchtungsvorrichtung und ein Verfahren zum Betreiben einer Beleuchtungsvorrichtung für ein Endoskop zu schaffen, welches flexibel und kostengünstig herstellbar ist und darüber hinaus eine hohe Qualität der Ausleuchtung und somit eine hohe Qualität der aufgenommenen Bilder erlaubt.

Diese Aufgabe wird durch die Vorrichtung gemäß Anspruch 1 und durch ein Verfahren gemäß Anspruch 30 gelöst.

Ausführungsbeispiele der vorliegenden Erfindung beschreiben eine Beleuchtungsvorrichtung für eine Bilderfassungseinrichtung am distalen Ende eines Endoskops, das einen Beleuchtungsträger, der am distalen Ende des Endoskops angeordnet ist, und eine Vielzahl von Mikro-LEDs aufweist, wobei die Mikro-LEDs jeweils eine Hauptoberfläche, von der die Abstrahlung erfolgt, aufweisen, die eine maximale laterale Ausdehnung von weniger als 500 µm aufweist. Die Mikro-LEDs sind derart an dem Beleuchtungsträger angeordnet, dass bei einer elektrischen Anregung die Umgebung des distalen Endes des Endoskops zumindest abschnittsweise beleuchtet ist. Dabei können die Mikro-LEDs insbesondere Array-förmig angeordnet sein, wobei das Array flächenmäßig (2-dimensional) oder auch zeilenförmig (1-dimensional) ausgestaltet und die Fläche oder Zeile gekrümmt oder eben sein kann (beispielsweise Kugel-, Zylinder- oder Quader-förmig). Der Beleuchtungsträger mit den Array-förmig angeordneten Mikro-LEDs kann ebenfalls auf einer Außenwand am distalen Ende des Endoskops angeordnet sein.

Bei weiteren Ausführungsbeispielen ist der Array an einem Beleuchtungsfinger angeordnet, der ebenfalls quaderförmig, zylinderförmig oder auch kugelförmig ausgestaltet sein kann. Darüber hinaus können verschiedene Mikro-LEDs Licht in verschiedene Abstrahlrichtungen emittieren. Im Gegensatz zu konventionellen LEDs, weisen Mikro-LEDs u.a. eine wesentlich kleinere Abmessung auf, so dass beispielsweise eine laterale Ausdehnung der Licht-emittierenden Oberfläche bzw. senkrecht zu einer Abstrahlrichtung von höchstens 500 µm oder kleiner als 100 µm aufweisen kann. Die Anordnung der Mikro-LEDs kann beispielsweise dicht erfolgen, so dass zwei benachbarten Mikro-LEDs möglichst kleinem oder keinen Zwischenraum untereinander aufweisen oder der Zwischenraum kleiner als die laterale Ausdehnung entlang der Verbindung der zwei benachbarten Mikro-LEDs (oder nicht größer als das 5-fache der lateralen Ausdehnung ist). Um eine möglichst gleichförmige Ausleuchtung zu erreichen kann es günstig sein, möglichst viele Mikro-LEDs in diesem Sinne dicht anzuordnen bzw. Gruppen von Mikro-LEDs zu bilden, deren Mikro-LEDs innerhalb einer Gruppe dicht angeordnet sind, die Gruppen untereinander jedoch einen größeren Abstand aufweisen.

Erfindungsgemäß können somit mehrere, kleinste Leuchtdioden (möglichst viele, entsprechend den vorhandenen Platzverhältnissen) in eine Weise am distalen Ende des Endoskops oder an dem Beleuchtungsträger angebracht sein, um die endoskopisch betrachtete Szene optimal auszuleuchten. Die Mikro-LEDs können dabei fest fixiert, verschiebbar, koppelbar oder steckbar (mittels einer Steckverbindung zum Beispiel) am Beleuchtungsträger fixiert sein.

Da bei endoskopischen Anwendungen in engen Hohlräumen der Platzbedarf eine wichtige Rolle spielt, ist eine miniaturisierte Ausführung in Form von Mikro- LEDs von hervorragender Bedeutung. Die Verwendung miniaturiserter Bauformen erlaubt einerseits einen platzsparenden Aufbau, andererseits - bei vergleichbarem Platzbedarf - die Anbringung einer größeren Anzahl von Mikro-LEDs. Dies ist im Hinblick auf die erzielbare Gleichmäßigkeit der Ausleuchtung von entscheidender Bedeutung. Außerdem können Mikro-LEDs mit verschiedenen Farben und/oder Abstrahlrichtungen auf dem Träger angeordnet sein.

Um die Miniaturisierung optimal zu realisieren, können die Mikro-LEDs gemäß Ausführungsbeispielen der Erfindung direkt als Chip, ganz ohne handelsübliche Gehäuse mit standardisierten Bauformen, angebracht werden. Dazu werden die winzigen Mikro-LED-Halbleiter-Chips mit einer Größe im Bereich von wenigen Mikrometern bis zu wenigen 100 µm mittels Verbindungstechniken wie z.B. Löt- oder Klebe-Verbindung auf eine Trägerfolie (z.B. flexibel oder vorgeformt und wenige Mikrometer dick), die gleichzeitig die elektrische Verbindung realisieren kann, aufgebracht. Die Trägerfolie ihrerseits wird wiederum auf die mechanisch tragende Struktur des Endoskop-Rohres oder eines zusätzlichen Beleuchtungsträgers aufgebracht. Der Beleuchtungsträger kann z.B. ein "Aufsteck-Rohr", das über das eigentliche Endoskoprohr geschoben wird, oder ein Lichtfinger, der durch einen Instrumentenkanal des Endoskops geschoben wird, sein. Die Trägerfolie kann beispielsweise als eine flexible Membran (flexible Schaltungsplatine) ausgestaltet sein und darüber hinaus ein transparentes Material aufweisen. Dies ist insbesondere dann vorteilhaft, wenn der Träger ebenfalls transparent ist. Eine vorgeformte Ausgestaltung der Trägerfolie ist insbesondere dann vorteilhaft, wenn beispielsweise der Beleuchtungsträger oder das Endoskop ein starke Krümmung aufweist, so dass es zu einer Beschädigung der Folie oder der Mikro-LEDs kommen könnte, wenn eine nicht vorgeformte Trägerfolie verwendet würde.

Zur Strahlformung können, wenn erforderlich, Mikrolinsen zur Lichtbündelung oder Diffusor-Elemente zur Lichtstreuung vor einzelnen, einem Teil oder allen LED-Chips (Mikro-LEDs) angebracht werden. Mit einem Lack oder einer anderen, transparenten, geeigneten Vergussmasse können die Komponenten gegen Beschädigung (z.B. mechanisch infolge von Kollisionen oder chemisch infolge von aggressive Flüssigkeiten) geschützt werden. Besitzt die Vergussmasse geeignete opake, optische Eigenschaften, so kann sie gleichzeitig als Diffusor oder Bündelungselement dienen. Mittels der Vergussmasse kann im Weiteren eine glatte äußere Oberfläche erzeugt werden.

Eine weitere Möglichkeit mit einem geringeren Miniaturisierüngsgrad ist die Anbringung der Mikro-LEDs in handelsüblichen, aber miniaturisierten Gehäusebauformen wie z.B. SMD-Gehäuse (SMD = Surface Mounted Device, d.h. ein miniaturisierte Gehäusebauform für Elektronikkomponenten), die bis in den Sübmillimeterbereich reichen können. Dazu werden die SMD-Gehäuse der LEDs mittels geeigneter Verbindungstechniken, z.B. Löt- oder Klebe-Verbindung angebracht.

Bevorzugte Ausführungsbeispiele der vorliegenden Erfindung werden nachfolgend Bezug nehmend auf die beiliegenden Zeichnungen näher erläutert. Es zeigen:
Fig. 1a einen Beleuchtungsträger (Träger) mit einer Array-förmigen Anordnung von Mikro-LEDs gemäß einem Ausführungsbeispiel der vorliegenden Erfindung;
Fig. 1b-d eine Mikro-LED an einem Träger, mit einer Linse und einem Diffusor;
Fig. 2 ein Träger mit einer Array-förmigen Anordnung von Mikro-LEDs gemäß einem weiteren Ausführungsbeispiel;
Fig. 3 eine zeilenförmige Anordnung von Mikro-LEDs an einem distalen Ende eines Endoskops;
Fig. 4 eine seitliche Anordnung von Mikro-LEDs an einem Endoskop;
Fig. 5 ein sogenannter Rundum-Retroblick für ein Endoskop mit Mikro-LEDs an zumindest einer Seitenwand;
Fig. 6 ein stabförmiger Träger für Mikro-LEDs;
Fig. 7 ein Endoskop mit einem beweglichen stabförmigen Träger für Mikro-LEDs;
Fig. 8 ein Endoskop mit einem beweglichen stabförmigen Träger für Mikro-LEDs außerhalb des Blickfelds der Optik;
Fig. 9 einen Träger für Mikro-LEDs an dem distalen Ende des Endoskops mit seitlichen Fenstern;
Fig. 10 Bilder von einer Anordnung von Mikro-LEDs mit und ohne Diffusor; und
Fig. 11 ein Ausleuchtungsbereich eines Trägers mit einem Array von Mikro-LEDs.

Bevor im Folgenden die vorliegende Erfindung anhand der Zeichnungen näher erläutert wird, wird darauf hingewiesen, dass gleiche Elemente oder gleichwirkende Elemente in den Figuren mit den gleichen oder ähnlichen Bezugszeichen versehen sind, und dass eine wiederholte Beschreibung dieser Elemente weggelassen wird bzw. Erläuterungen dieser Elemente in verschiedenen Figuren entsprechend aufeinander anzuwenden oder austauschbar sind.

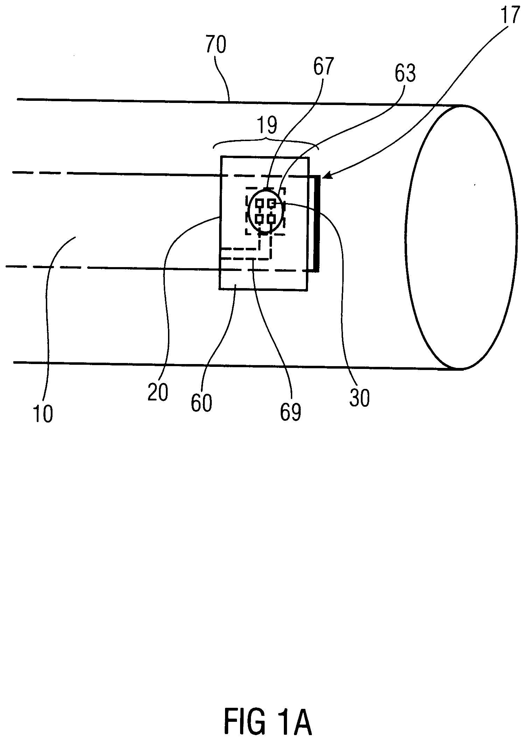

Fig. 1a zeigt eine Beleuchtungsvorrichtung 19 für eine Bilderfassungseinrichtung 17 am distalen Ende 20 eines Endoskops 10 mit einem Beleuchtungsträger 60 und einer Vielzahl von Mikro-LEDs 30 an dem Beleuchtungsträger 60 mit einer Hauptoberfläche, von der die Abstrahlung erfolgt und die eine maximale laterale Ausdehnung von weniger als 500 µm aufweist. Die Mikro-LEDs 30 sind so an dem Beleuchtungsträger 60 angeordnet, so dass bei elektrischer Anregung eine Umgebung des distalen Endes 20 des Endoskops 10 (z.B. das Innere eine Rohres 70) zumindest abschnittsweise zu beleuchten. In der Bilderfassungseinrichtung 17 erfolgt beispielsweise die optische Erfassung des Bildes, z.B. mittels einer Optik mit einem Objektiv. Dazu kann die Bilderfassungseinrichtung 17 beispielsweise Glasfaser (zur optischen Weiterleitung eines Bildes) oder auch Relais-Linsen aufweisen. Ferner kann das Endoskop 10 als ein Videoskop ausgelegt sein, so dass eine elektrische oder elektronische Wandlung des optischen Bildes bereits am distalen Ende 20 stattfinden kann.

Die Vielzahl von Mikro-LEDs 30 kann als ein Array 63 oder auch zeilenförmig auf einer Außenfläche des Beleuchtungsträgers 60 angeordnet sind und der Beleuchtungsträger 60 kann zumindest 10 Mikro-LEDs 30 aufweisen. Bei Ausführungsbeispielen der vorliegenden Erfindung weist die Haupt- bzw. Abstrahloberfläche eine maximale laterale Ausdehnung von weniger als 300 µm, 100 µm oder 10 µm auf und/oder die Hauptoberfläche einer Mikro-LED 30 weist einen Flächeninhalt von höchsten 0,01 mm² auf. Gemäß Ausführungsbeispielen der vorliegenden Erfindung kann der Beleuchtungsträger 60 mit dem distalen Ende 20 des Endoskops 10 mechanisch verbindbar (z.B. ankoppelbar) und/oder verschiebbar an denselben angeordnet sein, wobei der Beleuchtungsträger 60 aber auch durch einen Abschnitt des Endoskops 10 an dem distalen Ende 20 desselben ausgebildet sein kann.

Das Rohr 70 kann auch eine Teil einer Bohrung sein, die nur eine Öffnung, in die das Endoskop 10 eingeführt werden kann, aufweist. Die Mikro-LEDs 30 können weiterhin auch auf einen Folienträger 67 mit Leiterbahnen 69, die der elektrischen Kontaktierung (Energieversorgung) der Mikro-LEDs 30 dienen, angeordnet sein.

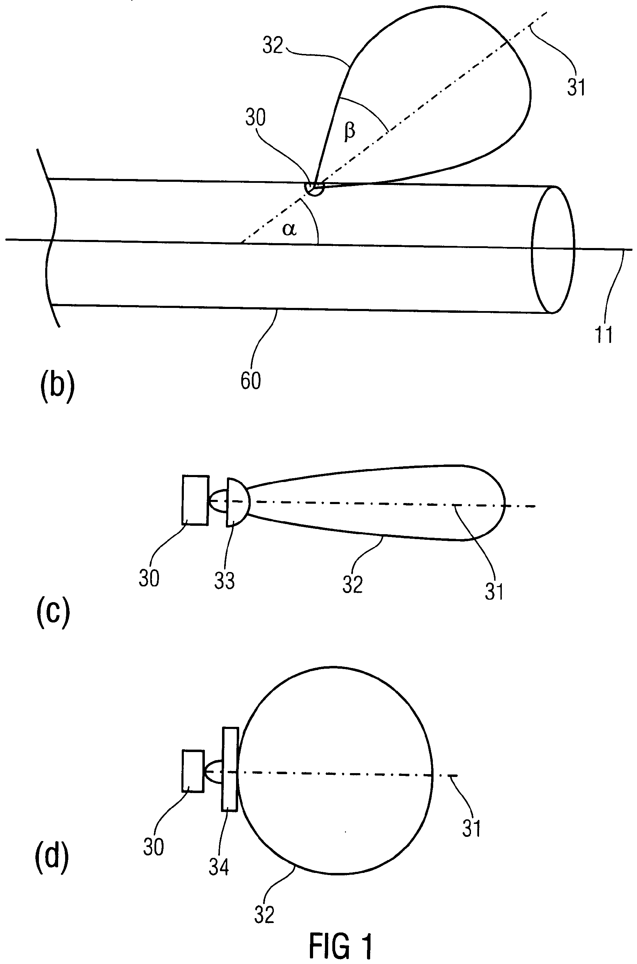

Fig. 1b zeigt einen Träger 60 für eine Mikro-LED 30, der ringförmig um eine geometrische Achse 11 einer Optik eines Endoskops 10 angeordnet ist. Die geometrische Achse 11 (oder Geräteachse) kann auch mit der optischen Achse eines Objektivs übereinstimmen (beispielsweise für starre Endoskope). Andererseits kann z.B. für flexible Endoskope, die das Bildfeld ablenken (z.B. mittels eines Prisma oder einer Linse bzw. mehrerer Linsen), die optische Achse sich von der geometrischen Achse 11 unterscheiden. Die Mikro-LED 30 weist eine Abstrahlrichtung 31 auf, und die Abstrahlung erfolgt in einer bestimmten Abstrahlcharakteristik 32. Die Abstrahlrichtung 31 weist dabei einen Winkel α zur optischen Achse 21 oder zur Hauptoberfläche der Mikro-LED 30, von der die Abstrahlung erfolgt, auf. Die Abstrahlcharakteristik 32 wird beispielsweise durch einen Öffnungswinkel β beschrieben, der angibt, in welchem Richtungsbereich die Mikro-LED 30 hauptsächlich abstrahlt (z.B. den Bereich, in welchem die Mikro-LED 30 zumindest 70% der Lichtintensität abstrahlt). Die Abstrahlrichtung 31 kann beispielsweise durch eine Optik (z.B. durch einen Spiegel) oder eine Variation der Form der Hauptoberflächen und/oder der Brechzahldifferenz zwischen der Hauptoberfläche und einem angrenzenden Mediums beeinflusst werden. Außerdem können die Mikro-LEDs 30 geneigt auf dem Träger angeordnet werden. Die Abstrahlcharakteristik 32 kann beispielsweise dadurch verändert werden, dass die Mikro-LED 30 zusätzliche eine Linse bzw. Zerstreuer (Diffusor) aufweist.

Fig. 1c-d zeigen eine Veranschaulichung, wie durch eine Mikrolinse 33 oder einen Diffusor 34 die Beleuchtungscharakteristik 32 verändert werden kann. In Fig. 1c ist eine Mikro-LED 30 gezeigt, die eine Mikrolinse 33 aufweist, so dass die Beleuchtungscharakteristik 32 einen kleineren Winkelwert β aufweist. Somit erfolgt eine verstärkte Abstrahlung hin zur Achse 31 (Fokussierung). Bei weiteren Ausführungsbeispielen ist eine Linse über mehrere Mikro-LEDs 30 angeordnet, so dass das Licht mehrerer Mikro-LEDs 30 gebündelt wird. Außerdem können alle oder auch nur ein Teil der Mikro-LEDs 30 jeweils eine Mikrolinse 33 aufweisen. Fig. 1d zeigt die Mikro-LED 30 mit einem Diffusor 34, so dass die Abstrahlcharakteristik 32 einen größeren Winkelwert für β aufweist. Somit wird verstärkt Licht weg von der Achse 31 abgestrahlt und es erfolgt somit eine Ausleuchtung eines breiteren Blickfeldbereiches. Auch hier kann ein Diffusor mehrere Mikro-LEDs 30 umfassen, oder ein Teil oder alle Mikro-LEDs 30 können jeweils einen Diffusor 34 aufweisen.

Bei Ausführungsbeispielen der vorliegenden Erfindung werden eine Vielzahl von Mikro-LEDs 30 zur Ausleuchtung genutzt. Zur Unterscheidung verschiedener Mikro-LEDs 30, die in verschiedene Richtungen 31 abstrahlen können und darüber hinaus verschiedene Abstrahlcharakteristiken 32 aufweisen können, wird folgende Notation benutzt. Mit dem Bezugszeichen aa.b.i wird im Folgenden eine Mikro-LED, wenn aa=30, eine Abstrahlrichtung, wenn aa=31, und eine Abstrahlcharakteristik, wenn aa=32, bezeichnet. Die Abstrahlcharakteristik 32 bezieht sich dabei beispielsweise auf eine fokussierte Abstrahlung, beispielsweise in Folge einer Mikrolinse, die auf die Mikro-LED 30 aufgebracht ist (siehe Fig. 1c), oder auf eine diffuse Abstrahlung, beispielsweise, wenn ein Diffusor 34 auf die Mikro-LED 30 aufgebracht ist (siehe Fig. 1d). Die Abstrahlrichtung 31 bezieht sich dabei auf jene Richtung, in der die Lichtintensität der Mikro-LED 30 ein Maximum aufweist. Der Wert b nummeriert verschiedene Abstrahlrichtungen 31 der Mikro-LEDs 30 bezüglich der Oberfläche des Trägers 60 durch. Schließlich bildet der Wert i eine Durchnummerierung von Mikro-LEDs 30, die eine gleiche Abstrahlrichtung 31 aufweisen.

Verschiedene Abstrahlrichtungen 31 beziehen sich dabei auf verschiedene Winkel α zwischen der Abstrahlrichtung und der Oberfläche des Trägers 60. Neben Abstrahlrichtungen parallel zur optischen Achse 21 (α = 0, 180°) kann der Winkel α größer als 15° oder größer als 25° sein. Für den Fall, dass fünf Abstrahlrichtungen 31 vorliegen, können die verschiedenen Abstrahlrichtungen 31 beispielsweise von 1 bis 5 durchnummeriert sein, so dass b=1, eine Abstrahlrichtung 31 parallel zur optischen Achse 21 weg von dem distalen Ende 20 (das eine Optik aufweisen kann) des Endoskops 10 bezeichnet (α=0°), eine Abstrahlrichtung 31 mit dem Wert b=3 eine Abstrahlrichtung 31 senkrecht zur optischen Achse 21 bezeichnet (α=90°), und eine Abstrahlrichtung 31 mit dem Wert b=5, eine Abstrahlrichtung 31 parallel zur optischen Achse 21 bezeichnet und zwar eine Abstrahlrichtung 31, die dem distalen Ende 20 des Endoskops 10 zugewandt ist (α=180°). Dementsprechend bezeichnet eine Abstrahlrichtung 32 für den Wert b=2 eine Richtung, für die der Winkel α einen Wert zwischen 0° und 90° (oder zwischen 10° und 90°, 30° und 60°, 40° und 50°) aufweist (z.B. α=45°) und b=4 eine Richtung, für die der Winkel α einen Wert zwischen 90° und 180° aufweist (z.B. α=135°). Bei den hier angegebenen Werten kann beispielsweise eine Toleranz von +/- 10° auftreten bzw. im Allgemeinen werden die Winkel entsprechenden eines gewünschten Blickwinkel angepasst werden können. In jeder Abstrahlrichtung 31 können mehrere Mikro-LEDs 30 abstrahlen, zum Beispiel zumindest 5 oder 10 Mikro-LEDs 30 pro Abstrahlrichtung 31, die für jede Abstrahlrichtung 31 auch unterschiedlich gewählt sein können.

Bei weiteren Ausführungsbeispielen sind die Mikro-LEDs 30 in mehr als fünf Abstrahlrichtungen 31 angeordnet. In einem Fall mit n weiteren Abstrahlrichtungen 31 (zusätzlich zu der Vorrausrichtung, α=0), können die Abstrahlrichtungen 31 beispielsweise so gewählt sein, dass die Differenz Δα zwischen zwei benachbarten Abstrahlrichtungen 180°/n beträgt. Andererseits können die Abstrahlrichtungen 31 auch flexibel den Erfordernissen angepasst werden.

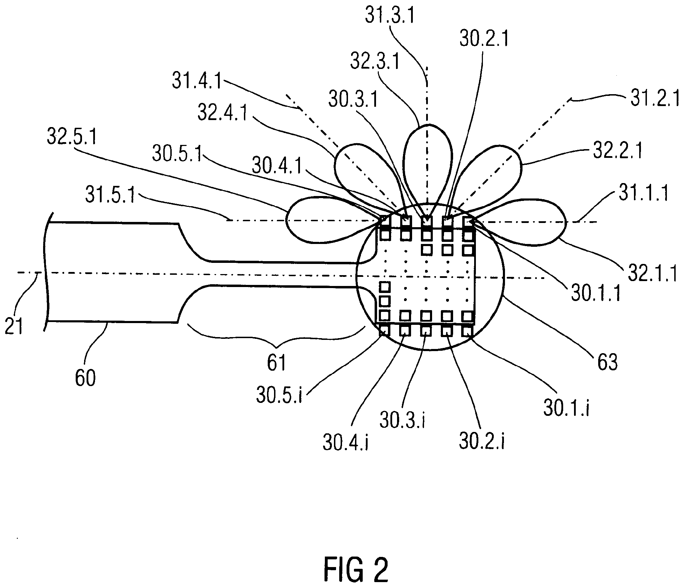

Fig. 2 zeigt einen Träger 60 mit einer Array-förmigen Anordnung 63 von Mikro-LEDs 30, die in verschiedene Richtungen abstrahlen. Konkret weisen die Mikro-LED 30 fünf Abstrahlrichtungen für die Werte b=1, 2, 3, 4, 5 auf. Der Wert b=1 entspricht dabei z.B. einem Winkel α=0, der Wert b=2 entspricht einem Wert α=45°, der Wert b=3 entspricht einem Wert α=90°, der Wert b=4 entspricht einem Wert α=135° und der Wert b=5 entspricht einem Wert α=180°, wobei die Angaben für den Winkel α eine Toleranz von beispielsweise ±20% aufweisen können. In einer ringförmigen Anordnung um die optische Achse 21 herum sind an dem Träger 60 in der Fig. 2 eine Vielzahl von Mikro-LEDs 30.b.i angeordnet, wobei b=1, 2, 3, 4, 5 und i=1,2,...,n eine Durchnummerierung der Mikro-LEDs 30 darstellt (n=Anzahl der Mikro-LEDs 30).

Der Träger 60 aus der Fig. 2 weist ferner einen Öffnungsbereich 61 auf, der derart ausgelegt ist, dass, wenn der Träger 60 auf ein Endoskop oder Endoskopende 10 aufgeschoben wird, eine Optik des Endoskops 10 eine Bilderfassung über den Öffnungsbereich 61 möglich ist. Die Optik kann dabei beispielsweise dazu dienen, das Bild auf eine Bilderfassungseinrichtung (einer Photo- oder Videokamera, CCD Kamera) zu projizieren oder das optische Bild vom distalen Ende zum proximalen Ende zu übertragen (z.B. mittels eines Linsensystems oder auch eines Lichtleiters).

Der Beleuchtungsträger 60 kann dabei mit dem Endoskop 10 über eine Steckverbindung unter Verwendung eines Steckers, der gleichzeitig die elektrischen Zuleitungen bereitstellen kann, verbunden werden. Andererseits kann der Beleuchtungsträger 60 auch elektrisch (mittels Servomotoren beispielsweise) oder mechanisch verschiebbar an dem Endoskop 10 angeordnet sein. Der Beleuchtungsträger 60 kann schließlich auch fest (z.B. mittels einer Klebeverbindung) mit dem Endoskop 10 verbunden werden.

Die Abstrahlcharakteristik 32 der Beleuchtung kann beispielsweise durch unterschiedliche Orientierung der Mikro-LEDs 30 (LED-Chips) an eine Blickrichtung des Endoskops 10 sowie an eine Prüfaufgabe (Anwendungsgebiet) angepasst werden. Beispielsweise können durch separates Ein- und Ausschalten der entsprechenden Mikro-LEDs 30, jene Mikro-LEDs, die nach vorne (b=1), schräg zur Seite (b=2), zur Seite (b=3), schräg nach hinten (b=4) oder nach hinten (b=5) strahlen, aktiviert werden. Darüber hinaus sind auch beliebige, sinnvolle Kombinationen von Abstrahlrichtungen 31 möglich. Die in der Fig. 2 dargestellten Mikro-LEDs 30 sind nur exemplarisch gezeichnet. Die Anzahl als auch eine Packungsdichte kann entsprechend den vorhandenen Platzverhältnissen so groß wie möglich gewählt werden, um dadurch die Ausleuchtung der Umgebung so optimal wie möglich zu gestalten, z.B. können zumindest 10, 20, 50 oder 60 Mikro-LEDs 30 an einem Beleuchtungsträger 60 angeordnet sein. Wenn beispielsweise der Beleuchtungsträger 60 (z.B. einen Lichtfinger) einen Umfang von 2 mm Durchmesser aufweist können ca. 60 Mikro-LEDs 30 mit einer Abmessung von je 100 µm angebracht werden.

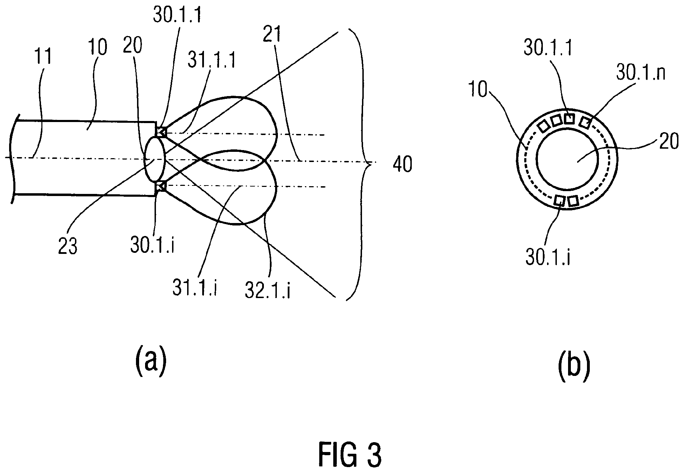

Fig. 3 zeigt eine zeilenförmige Anordnung von Mikro-LEDs 30 am distalen Ende 20, das beispielsweise ein Objektive 23 aufweisen kann, herum, wobei bei diesem Ausführungsbeispiel alle Mikro-LEDs 30 in eine Vorausrichtung (b=1) abstrahlen. Beim Vorausblick ist die optische Achse 21 des Objektivs identisch mit der mechanischen Achse 11 des Endoskoprohres 10 am distalen Ende 20.

Am distalen Ende 20 des Endoskops 10 sind um das Objektiv 23 ringförmig, dicht gepackt, nach vorne strahlende Mikro-LEDs 30.1.1 bis 30.1.n mit der Abstrahlrichtung 31.1 sowie den Intensitätsverteilungen 32.1 angebracht, die das beobachtbare Sichtfeld 40 ausleuchten. Vorteilhaft ist einerseits der erheblich höhere Miniaturisierungsgrad, so dass auch wesentlich kleinere, dünnere Geräte, die zur Untersuchung auch sehr kleiner Hohlräume geeignet sind, realisiert werden können. Andererseits kann dadurch eine Anordnung mit einer relativ großen Anzahl von Mikro-LEDs 30 von mehr als 10 realisiert werden. So können am distalen Ende 20 rund um das Objektiv 23 eines 3mm Endoskops 10 ca. 50 - 60 Mikro-LED-Chips 30 platziert werden. Dadurch kann eine erheblich verbesserte Qualität der Ausleuchtung erreicht werden sowie durch individuelle Ansteuerung einzelner Mikro-LEDs 30 oder Mikro-LED-Gruppen (z.B. in verschiedenen Farben) eine große Zahl unterschiedlicher Beleuchtungsmodalitäten (hell, dunkel, verschiedenfarbig, etc.) realisiert werden.

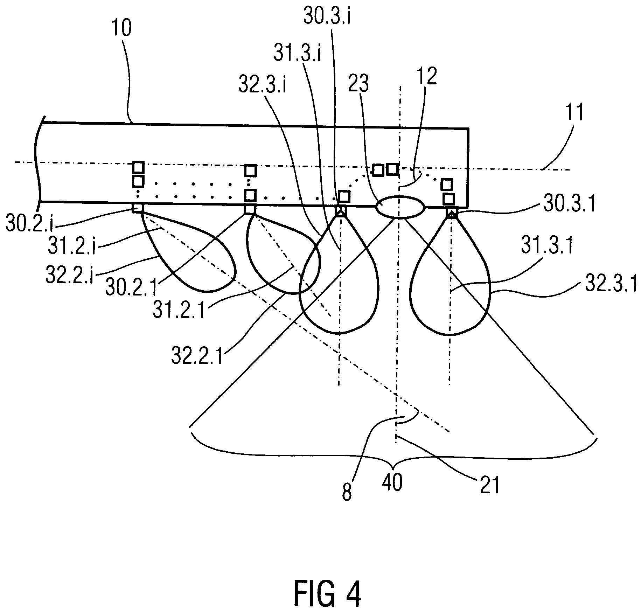

Fig. 4 zeigt eine Anordnung von Mikro-LEDs, die seitlich an ein Endoskoprohr 10 angeordnet sind, so dass für ein seitlich angeordnetes Objektiv 23 des Endoskops 10 mit einem Blickfeld 40 eine Beleuchtung bereitgestellt wird. Beim Seitblick bildet die optische Achse 21 des Objektivs 23 einen Winkel 12 mit der mechanischen Achse 11 des Endoskoprohres 10 am distalen Ende. Mit der Summe des Winkels 12 und des Winkels α kann beispielsweise ein Winkel γ gebildet werden, der den Winkel zwischen der optischen Achse 21 und der Abstrahlrichtung 31 darstellt und die optische Achse 21 beispielsweise geradeaus, seitwärts oder schräg nach hinten gerichtet sein kann. Der Winkel 12 und der Winkel γ können somit einen Wert zwischen 0° (nach vorne) und +/- 160° (nach hinten) aufweisen.

Bei diesem Ausführungsbeispiel sind die Mikro-LEDs 30 in verschiedene Gruppen, 30.2.1 - 30.2.i bis 30.3.1 - 30.3.j angebracht, die sich durch unterschiedliche Abstrahlrichtungen 31.2.1 - 31.2.i bis 31.3.1 - 31.3.j auszeichnen und jeweils das beobachtbare Bildfeld 40 ausleuchten. Die verschiedenen Mikro-LEDs 30 bzw. Mikro-LED-Gruppen können durch individuelle Ansteuerung in ihrer Helligkeit geregelt werden, so dass dadurch unterschiedliche Beleuchtungsmodalitäten realisiert werden können. Die zylindrische Wandung einer Bohrung oder eines Hohlraums kann damit immer durch alleiniges Umschalten der Leuchtstärke der verschiedenen Mikro-LEDs 30 bzw. Mikro-LED-Gruppen in einer optimalen Hellfeld- oder Dunkelfeld-Anordnung ausgeleuchtet werden.

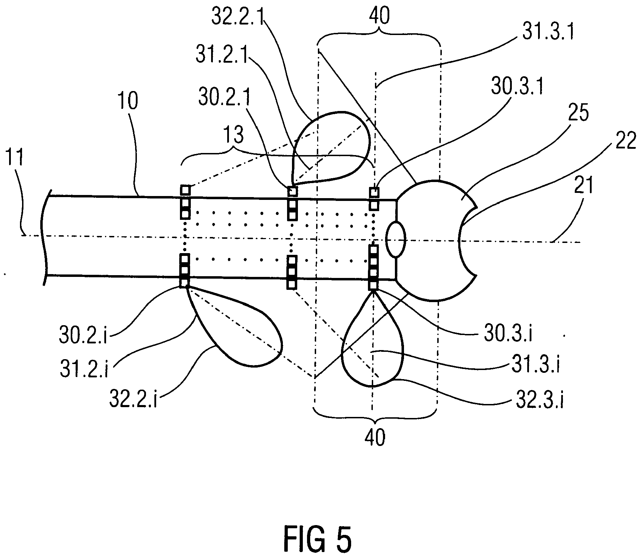

Fig. 5 zeigt ein Ausführungsbeispiel der vorliegenden Erfindung, bei dem am distalen Ende 20 des Endoskops 10 eine Linse 25 aufweist, die einen Rundum-Retroblick oder Rundumbilderfassung erlaubt (z.B: ist dies möglich mit einer sogenannten Greguss-Linse oder einem Kegelspiegel) und das Endoskoprohr 10 weist eine Array-förmige Anordnung von Mikro-LEDs 30 an der Außenwand in einem Bereiche 13 auf, wobei bei diesem Ausführungsbeispiel verschiedene Abstrahlrichtungen 31 für verschiedene Mikro-LEDs 30 gewählt wurden.

Beispielsweise können dies die Abstrahlrichtungen b=2, b=3 sein. Beim Rundum-Retroblick ist die optische Achse 21 der Linse 25 (z.B. "Greguss-Linse") identisch mit der mechanischen Achse 11 des Endoskoprohres 10 am distalen Ende. Durch einen Frontspiegel 22 wird das beobachtbare Bildfeld 40 so umgelenkt, dass ein Rundumblick seitlich oder schräg nach hinten erfolgt, aber kein Blick nach vorne möglich ist.

Die Mikro-LEDs 30 sind in verschiedenen Gruppen, 30.2.1 - 30.2.i bis 30.3.1 - 30.3.j angebracht, die sich durch unterschiedliche Abstrahlrichtungen 31.2.1 - 31.2.i bis 31.3.1 - 31.3.j aufweisen und jeweils das beobachtbare Bildfeld 40 ausleuchten. Die verschiedenen Mikro-LEDs 30 bzw. Mikro-LED-Gruppen können durch individuelle Ansteuerung in ihrer Helligkeit geregelt werden, so dass dadurch unterschiedliche Beleuchtungsmodalitäten realisiert werden können. Die zylindrische Wandung einer Bohrung oder eines Hohlraums kann damit durch Umschalten der Leuchtstärke der verschiedenen Mikro-LEDs 30 bzw. Mikro-LED-Gruppen in einer optimalen Hellfeld- oder Dunkelfeld-Anordnung ausgeleuchtet werden.

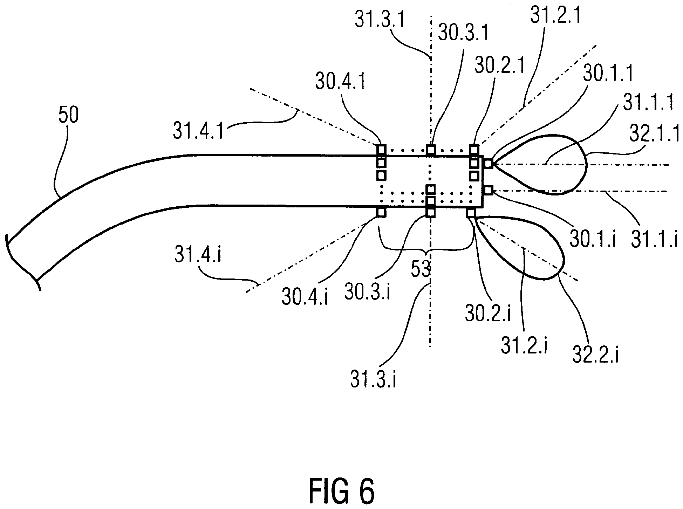

Fig. 6 zeigt ein Ausführungsbeispiel, bei dem eine Array-förmige Anordnung von Mikro-LEDs 30 in einem Bereich 53 eines Lichtfingers 50 angeordnet sind. Der Lichtfinger 50 ist ein Beispiel für einen separaten Beleuchtungsträger, bei dem die Mikro-LEDs 30 in analoger Weise zu dem Endoskoprohr 10 im Wesentlichen zylindrisch angeordnet werden. Die Abstrahlrichtungen 31 für verschiedene Mikro-LEDs 30 sind bei dem Ausführungsbeispiel von Fig. 6 dabei 31.1, 31.2, 31.3 und 31.4. Bei weiteren Ausführungsbeispielen sind jedoch noch weitere Richtungen oder auch Kombinationen möglich.

Der Lichtfinger 50 kann so geformt sein, dass er durch einen Instrumentenkanal 52 des Endoskops 10 vom proximalen zum distalen Ende 10 (nicht in der Fig. 6 gezeigt) vorgeschoben werden kann und dort austritt. Der als Lichtfinger 50 ausgestaltete starre oder flexible stabförmiger (zylindrischer) Körper trägt dabei an der distalen Spitze die Mikro-LEDs 30.

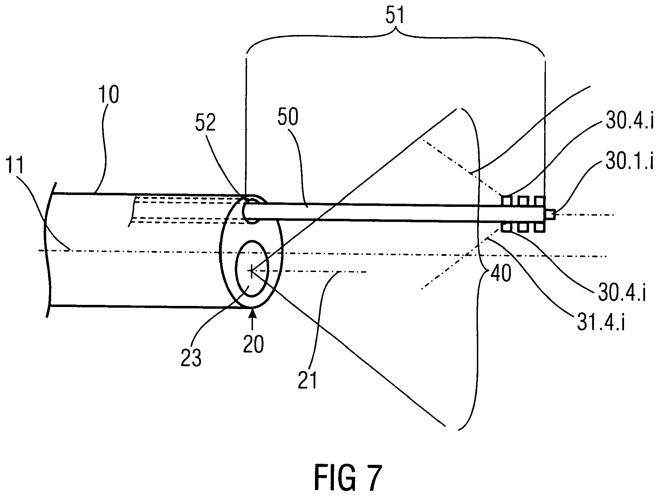

Fig. 7 zeigt ein Ausführungsbeispiel, bei dem die Mikro-LEDs 30, wie in Fig. 6 gezeigt, auf einem Lichtfinger 50 angeordnet sind, wobei der Lichtfinger 50 beweglich in einem Instrumentenkanal 52 des Endoskops 10 angebracht ist und beispielsweise durch ein Ein- und Ausschieben des Lichtfingers 50 verschiedene Bereiche beleuchtet werden können. Bei diesem Ausführungsbeispiel weist das Endoskop 10 ein Objektiv 23 mit einem Blickfeld 40 am distalen Ende 20 auf, wobei die optische Achse 21 von der geometrischen Achse 11 verschoben ist.

Damit ist eine Realisierung einer Hellfeld-Beleuchtung zylindrischer Bohrungswandungen in einer Gegenlicht-Anordnung möglich, wobei dazu der Lichtfinger 50 um ein gewisses Maß 51 vor die Spitze des Endoskops 10 vorgeschoben wird, so dass durch Aktivierung der nach hinten (Richtungen 31.3 - 31.5) strahlenden Mikro-LEDs 30 eine Hellfeld-Beleuchtung realisiert wird. Durch Variation des Maßes 51 kann dabei die Beleuchtungsqualität noch beeinflusst und optimiert werden. Die bei vorgeschobenem Lichtfinger 50 zwangsläufig entstehenden Verdeckungseffekte können beispielsweise durch eine Drehung zwischen dem Ein- und Ausschieben des Endoskops 10 kompensiert werden.

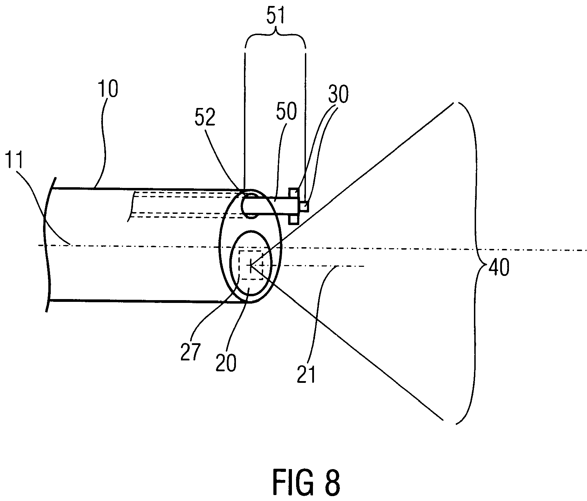

Fig. 8 zeigt ein Ausführungsbeispiel, bei dem das Endoskop 10 wie in Fig. 7 einen beweglichen Lichtfinger 50 mit Mikro-LEDs 30 aufweist, wobei der Lichtfinger 50 bei dem in Fig. 8 gezeigten Ausführungsbeispiel soweit in den Instrumentenkanal 52 des Endoskops 10 hineingeschoben wurde, dass der Blickbereich 40 des Objektivs 23 des Endoskops 10 nicht durch den Lichtfinger 50 eingeschränkt wird. Dabei kann der Lichtfinger 50 soweit zurückgezogen werden, dass das Maß 51 so klein wird, dass keine Verdeckungseffekte im beobachtbaren Bildfeld 40 auftreten. Werden die nach vorne (31.1 - 31.3) strahlenden Mikro-LEDs 30 aktiviert, so kann für den Boden einer Sackbohrung eine Hellfeld-Beleuchtung in Auflicht-Anordnung realisiert werden.

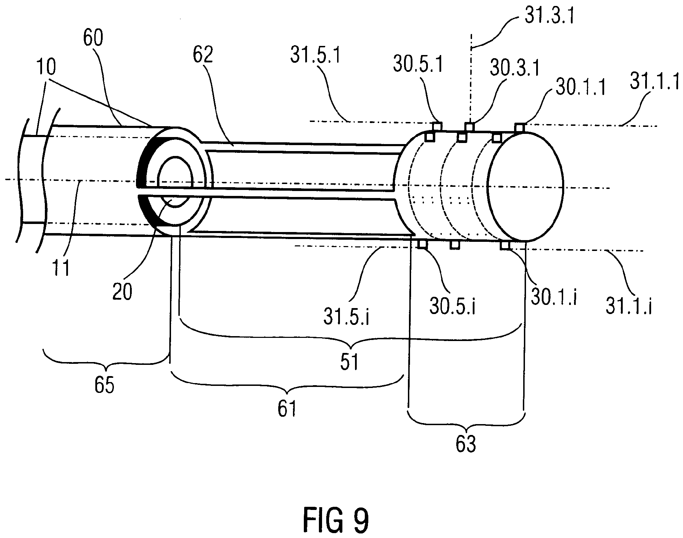

Fig. 9 zeigt ein Ausführungsbeispiel, bei dem der Träger 60 für die Mikro-LEDs 30 als ein dünnwandiges Rohr ausgebildet ist, welches einen Fensterbereich 61 aufweist. Der Fensterbereich 61 ermöglicht somit, dass das Objektiv 23 am distalen Ende 20 des Endoskops 10 das beleuchtete Umfeld erfassen kann. Bei diesem Ausführungsbeispiel stimmt die geometrische Achse 11 mit der optischen Achse 21 überein. Die Mikro-LEDs 30 können mit Abstrahlrichtungen 31.1 bis 31.5 oder in beliebigen Kombinationen dieser Richtungen angebracht sein. Das Rohr 60 ist so geformt, dass es passend über das distale Ende 20 des Endoskops 10 geschoben werden kann. Das (Aufsteck-) Rohr 60 ist in drei Bereiche unterteilt, wobei der Fensterbereich 61 einen Bereich 63, der die Mikro-LEDs 30 aufweist, von einem Haltebereich 65, der auf das Endoskop 10 aufgeschoben wird, trennt. Das Maß 51 von Fig. 7 und Fig. 8 umfasst somit den Fensterbereich 61 und den Bereich 63. Der Fensterbereich 61 weist beispielsweise große Fenster über den gesamten Umfang in das Rohr 60 auf; so dass in dem Fensterbereich 61 nur noch schmale Stege 62 bestehen, und so ein weitgehend ungehinderter Blick 40 vom Inneren des Aufsteckrohres nach außen möglich ist. Die elektrische Versorgung der Mikro-LEDs 30 kann entlang der Stege 62 geführt werden. Optional kann sowohl der Haltebereich 65 als auch die Stege 62 weitere Mikro-LEDs 30 aufweisen, die ebenfalls in verschiedene Abstrahlrichtungen 31 abstrahlen können, d.h. die entsprechend allen beschriebenen Ausführungsbeispielen angeordnet bzw. ausgebildet sein können.

Zur Realisierung einer Hellfeld-Beleuchtung zylindrischer Bohrungswandungen in einer Gegenlicht-Anordnung wird das Aufsteckrohr 60 dabei um ein gewisses Maß 51 vor die Spitze des Endoskops 10 vorgeschoben, so dass durch Aktivierung der nach hinten (31.3 - 31.5) strahlenden Mikro-LEDs 30 eine Hellfeld-Beleuchtung realisiert wird. Durch Variation des Maßes 51 kann dabei die Beleuchtungsqualität noch beeinflusst und optimiert werden. Die bei vorgeschobenem Aufsteckrohr zwangsläufig entstehenden Verdeckungseffekte können wie bereits beschrieben beispielsweise durch eine oder mehrere Drehungen kompensiert werden.

Werden die nach vorne (31.1 - 31.3) strahlenden Mikro-LEDs 30 aktiviert, so kann für den Boden einer Sackbohrung eine Hellfeld-Beleuchtung in Auflicht-Anordnung realisiert werden. Dabei kann das Aufsteckrohr 60 so zurückgezogen werden, dass das Maß 51 so klein wird, dass keine Verdeckungseffekte im beobachtbaren Bildfeld 40 auftreten.

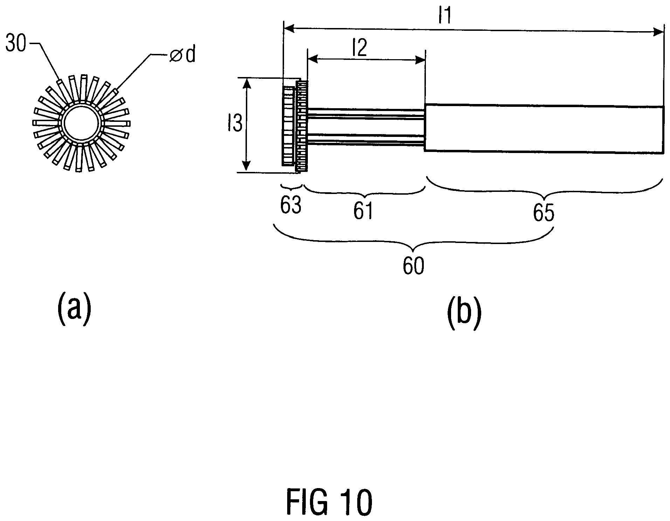

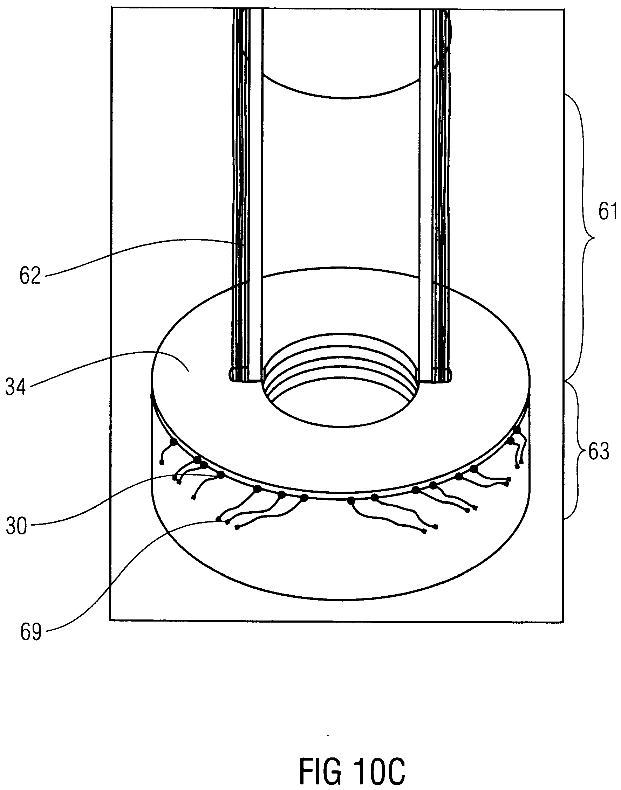

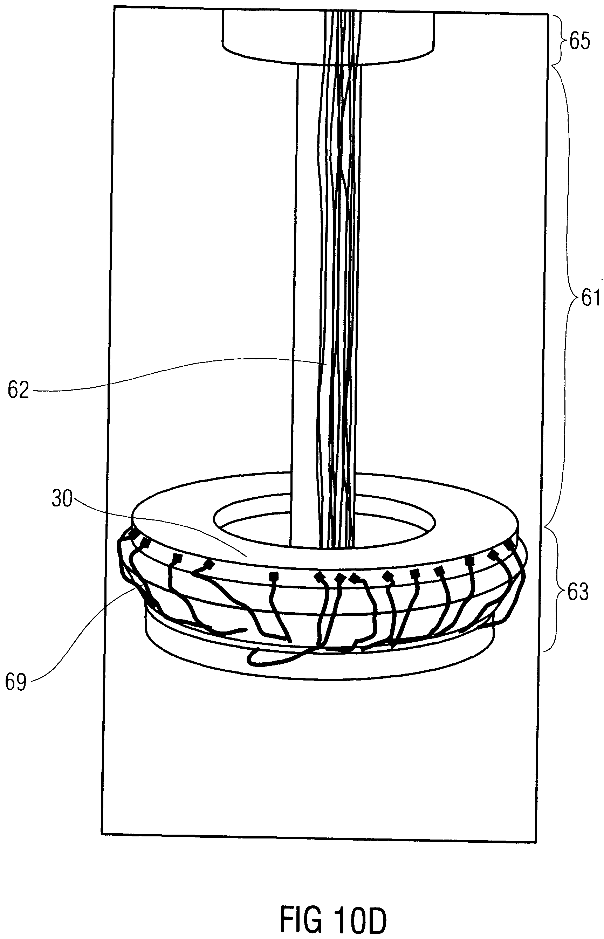

Fig. 10 zeigt ein Ausführungsbeispiel, bei dem eine ringförmige Anordnung von Mikro-LEDs 30 einen Diffusor 34 aufweisen. Fig. 10a zeigt dabei eine Frontansicht einer Anordnung von 33 Mikro-LEDs 30, die ringförmig um einen Kreis mit einem Durchmesser d angeordnet sind. Fig. 10b zeigt eine Seitenansicht eines Trägers 60, der an einem Ende 63 die ringförmige Anordnung der Mikro-LEDs 30 wie in Fig. 10a gezeigt, aufweist. Weiterhin weist das Ausführungsbeispiel von Fig. 10b einen Fensterbereich 61 auf, der eine Bilderfassung durch ein Endoskop 10, das in den Träger 60 in dem Bereich 65 eingeschoben wird, erlaubt. Beispielhafte Abmessungen für den Träger 60 umfassen eine Gesamtlänge 11 von ca. 64 mm, für den Öffnungsbereich 61 eine Länge 12 von ca. 20 mm und für einen Durchmesser 13 der ringförmigen Anordnung der Mikro-LEDs 30 von ca. 15,7 mm, wobei diese Abmessungen eine Toleranz von +/- 50% aufweisen können.

Das Aufsteckrohr 60 kann beispielsweise insgesamt 52 Mikro-LEDs 30 in zwei Abstrahlrichtungen, z.B. nach vorne (31.1) und nach hinten (31.5), aufweisen, wobei die Anzahl der Mikro-LEDs 30 für jede Abstrahlrichtung variieren kann. Das Aufsteckrohr 60 hat einen Innendurchmesser d von ca. 6,6 mm, passend für ein 6,5 mm Endoskop. Für beide Abstrahlrichtungen 31 ist jeweils eine Streuscheibe (Diffusor 34) vor den Mikro-LEDs 30 angebracht. Fig. 10c, d zeigen Abbildungen mit und ohne Diffusor-Elementen 34.

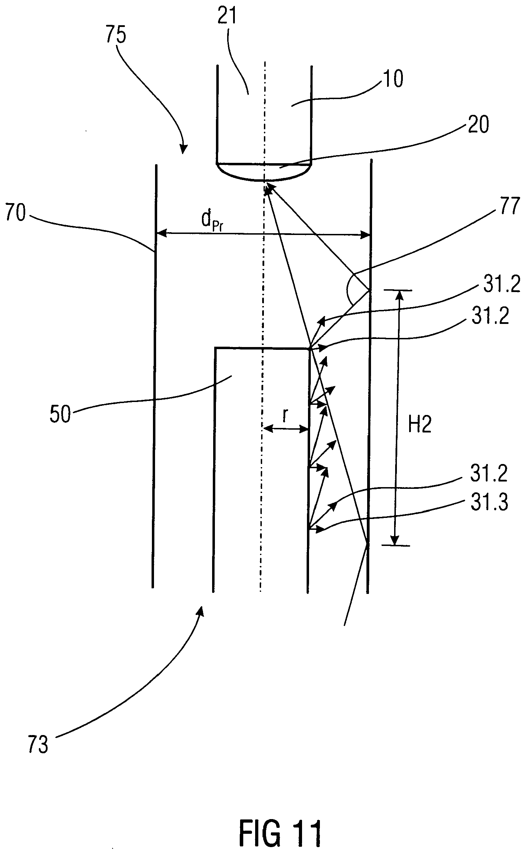

Fig. 11 zeigt ein Ausführungsbeispiel, bei dem ein Hohlraum im Innern eines Rohres 70, der eine erste Öffnung 73 und eine zweite Öffnung 75 aufweist, mit einem Endoskop 10 untersucht werden soll. Dazu wird der Träger 50 mit dem Mikro-LEDs 30 (in der Fig. 11 nicht gezeigt) von der ersten Öffnung 73 in den Hohlraum eingebracht. Der Träger 50 weist einen radialen Durchmesser r auf und hat an der Außenseitenwand Mikro-LEDs 30, die in verschiedenen Richtungen z.B. 31.2 und 31.3 abstrahlen. Über Reflexionen an der Innenwand des Rohres 70 gelangen die Lichtstrahlen 77 dann ins Objektiv 23 des Endoskops 10. Der Durchmesser des Rohres 70 beträgt d_{Pr} und die optische Achse 21 ist hier entlang es Mittelpunktes des Rohres 70 gelegt. Ein ausgeleuchtetes Feld ist dabei mit H2 bezeichnet, wobei beispielsweise die Entfernung H2 größer als d_{Pr} sein kann. Ein Vorteil der vorliegenden Erfindung ist dabei insbesondere dadurch gegeben, dass der Beleuchtungsbereich H2 durch verschiedene Abstrahlrichtungen 31.b deutlich größer gewählt werden kann, als dies für nur eine vorbestimmte Abstrahlrichtung 31 der Fall gewesen wäre.

Bei weiteren Ausführungsbeispielen erfolgt eine elektronische Bilderfassung direkt am distalen Ende 20 des Endoskops 10 (z.B. durch eine CCD-Kamera) oder das Bild wird durch optische Mittel (z.B. mittels eines Objektivs, Linsen, etc.) erfasst und an das proximale Ende weitergeleitet, wo eine Bilderfassung ebenfalls möglich ist. Die Weiterleitung kann dabei beispielsweise auch mittels eines Lichtleiters geschehen.

Zusammenfassend bietet die erfindungsgemäße Verwendung von Mikro-LEDs 30 somit verschiedenste Einsatzmöglichkeiten und ist insbesondere für die automatische Endoskopie vorteilhaft. Zum Beispiel kann mit einer einzigen Inspektion ("Befahrung") einer beispielhaften Sackbohrung die Seitenwand sowie der Boden der Bohrung 70 komplett mit optimaler Ausleuchtung erfasst werden. Für die Erfassung der Wandung befindet sich das Aufsteckrohr bzw. der Lichtfinger 50 in vorgeschobener Position, die (schräg) rückwärts strahlenden Mikro-LEDs 30 sind eingeschaltet: Die Bohrungswand wird in Hellfeld-Anordnung beleuchtet und aufgenommen. Am Ende der Sackbohrung wird das Aufsteckrohr bzw. der Lichtfinger 50 zurückgezogen, bis der Blick auf den Boden der Bohrung ungehindert ist, die vorwärtsstrahlenden Mikro-LEDs 30 werden eingeschaltet und der Boden der Bohrung kann erfasst werden.

Ebenfalls ist eine Verwendung von Mikro-LEDs 30 in verschiedenen Farben (zum Beispiel in Gruppen zusammengefasst) für viele Anwendungen vorteilhaft (da beispielsweise bestimmte Strukturen in bestimmten Farben besonders deutlich hervortreten). Außerdem bietet die flächenmäßige Abstrahlung mittels Arrays den Vorteil, dass eine gleichmäßige Ausleuchtung größerer Gebiete erreicht werden kann. Durch ein separates Ein- und Ausschalten bestimmter Gruppen von Mikro-LEDs 30 (die optional auch in verschiedene Farben) können weiterhin ganz gezielt bestimmte Gebiete beleuchtet werden. Schließlich kann ein gepulster Betrieb für eine automatische Bilderkennung sinnvoll sein, zum Beispiel zur Vermeidung von Bewegungsunschärfen. Die gepulste Ansteuerung kann sich dabei auf alle Mikro-LEDs 30 oder nur auf einen Teil der Mikro-LEDs beziehen.

Weitere Vorteile erfindungsgemäßer Ausführungsbeispiele können wie folgt zusammengefasst werden.

Durch die geringe Größe der LED-Chips, der Mikrolinsen 33 oder Diffusoren 34 sowie elektrischen Zuleitungen 69 im Bereich von wenigen Mikrometern bis zu wenigen 100 µm (z.B. von 1 µm bis 500 µm oder 3 µm bis 200 µm), kann ein extrem platzsparender Aufbau mit vergleichbarem oder kleinerem Volumen wie bei lichtleitenden Glasfasern erfolgen (insbesondere wichtig für sehr kleine Hohlräume, wesentlich kleiner als ca. 5-10mm, die mit dünnen Endoskopen untersucht werden sollen). Beispielsweise können Mikro-LEDs 30eine maximale laterale Ausdehnung einer Hauptoberfläche, von der die Abstrahlung erfolgt, von weniger als 500 µm, 300 µm oder 100µm und beispielsweise von 1 - 20 µm bezüglich einer Kantenlänge oder Diagonale aufweisen. Die Mikro-LEDs 30 können beispielsweise quaderförmig, würfelförmig oder auch eine längliche Gestalt aufweisen, wobei alle oder ein Teil der Seitenflächen die maximale laterale Ausdehnung aufweisen können.

Erfindungsgemäße Ausführungsbeispiele erlauben somit insbesondere eine Anordnung mit weit mehr als beispielsweise vier LEDs am distalen Ende des Endoskops, die beispielsweise ringförmig um die Pupille des Objektivs angeordnet sein können. Erfindungsgemäße Ausführungsbeispiele können somit, infolge der kleinen Abmaße der Mikro-LEDs 30 auch für kleine Bohrungen mit einem Durchmesser von 10 mm oder weniger verwendet werden. Es ist ebenfalls möglich, die Arbeitsspitze des Endoskops 10 mit einer Matrix von kleinen, superhellen LEDs in SMD-Bauform ausgestattet wird.

Aufgrund ihrer geringen Größe können die Mikro-LEDs 30 in nahezu beliebigen Orientierungen am distalen Ende 20 des Endoskops 10 angebracht werden, so dass unterschiedlichste Ausleuchtungscharakteristiken, je nach Anwendungsfall, realisiert werden können.

Aufgrund ihrer geringen Größe können die Mikro-LEDs 30 auch in mehreren unterschiedlichen Orientierungen am distalen Ende 20 des Endoskops 10 angebracht werden, so dass unterschiedliche Ausleuchtungscharakteristiken während einer Inspektion in einfacher Weise durch elektrisches Umschalten oder Dimmen der verschiedenen Mikro-LEDs 30 oder Mikro-LED-Gruppen realisiert werden können.

Durch Anbringung von vielen Mikro-LEDs 30 verteilt über einen gewissen Bereich des Endoskopschafts oder des externen Beleuchtungsträgers 50, kann ein größerer Bereich der inneren Oberfläche (des zu untersuchenden Hohlraumes) optimal ausgeleuchtet werden, so dass eine Inspektion schneller erfolgen kann. Ein Hin- und Herschieben des Beleuchtungsträgers 50 oder des Endoskops 10 kann dadurch vermieden werden.

Durch die Lichterzeugung direkt am distalen Ende 20 des Endoskops 10 entfällt die Lichtübertragung über verlustbehaftete, voluminöse Lichtleiter aus Glas- oder Kunststofffasern.

Die resultierende Lichtausbeute ist wesentlich höher, so dass Hoch- oder Höchstleistungs-LEDs nicht unbedingt erforderlich sind.

Die Energieversorgung mit elektrischer Energie kann durch sehr dünne Verdrahtungen oder dünne, flexible Leiterfolien 67 erfolgen. Dadurch können die Mikro-LEDs 30 einzeln oder in Gruppen vom proximalen Ende 20 aus mittels regelbarer elektrischer Stromversorgungen gesteuert werden.

Voluminöse, teure, externe Kaltlichtquellen, mit z. T. teuren, kurzlebigen Hochleistungslampen entfallen und können durch kompakte Stromversorgungsgeräte geringerer Leistung für die Mikro-LEDs 30 ersetzt werden.

Durch die nahezu verzögerungsfreie Steuerung der Lichtintensität durch den LED-Strom können gepulste oder stroboskopische Beleuchtungsmodalitäten realisiert werden, was insbesondere bei der Aufzeichnung bewegter Bildsequenzen mittels Kamera beim Befahren eines Hohlraumes von Bedeutung ist (Vermeidung von Bewegungsunschärfe).

Durch die Verfügbarkeit von weißen, verschiedenfarbigen oder mehrfarbigen Mikro-LED-Bauelementen 30 können bei geeigneter, kombinierter Anbringung verschiedener solcher Mikro-LEDs 30 endoskopische Beleuchtungsvorrichtungen 19 mit elektrisch steuerbarer Farbe, bzw. veränderbarem Farbton, einfach realisiert werden. Dies ist in Anwendungsfällen, bei denen die Farbe wesentlicher Untersuchungsgegenstand ist (z.B. Entzündungsstadium von menschlichem Gewebe) wichtig.

Schließlich kann durch Wahl der Abstrahlrichtung 31 (z.B. durch ein Zu- oder Abschalten von Mikro-LEDs 30 mit verschiedene Abstrahlrichtungen) das oben genannte Problem des nicht erfassbaren Gebietes gelöst werden.

Die Mikro-LEDs 30 können mit dem Endoskop 10 fest verbunden sein oder auf, relativ zum Endoskop 10 beweglichen, Trägern 60, 50 angebracht sein.

Ein Beleuchtungsträger 50, 60 kann auf verschiedener Art ausgestaltet sein, zum Beispiel als ein fester oder beweglicher Beleuchtungsträger 60, als ein Aufsteckrohr oder als ein Lichtfinger 50.

Bei einem festen Beleuchtungsträger 60 können die Mikro-LEDs 30 am oder nahe dem distalen Ende 20 des Endoskops 10 fest angebracht sein. Dabei können in einfacher Weise unterschiedliche Beleuchtungsmodalitäten realisiert werden, indem die Mikro-LEDs 30 mit unterschiedlichen Abstrahlrichtungen 31 oder -charakteristiken 32 angebracht werden und jeweils entsprechend der konkreten Situation / Aufgabenstellung durch eine Steuereinrichtung in ihrer jeweiligen Leuchtintensität gesteuert werden.

Eine Mikro-LED-Beleuchtung auf einem externen, gegenüber dem Endoskop 10 beweglichen Beleuchtungsträger 50, 60 ist dahingehend vorteilhaft, da vor Ort, bei eingeführtem Endoskop 10, die räumliche Anordnung der Beleuchtung relativ einem beobachtenden Objektiv, das sich oft am distalen Ende 20 befindet, verändert werden kann und so im Zuge einer einzigen Inspektion unterschiedliche Ausleuchtungen des Hohlraumes realisieren werden können.

Eine Verwendung von Lichtfingern 50 als ein externer Beleuchtungsträger ist andererseits vorteilhaft bei der Prüfung von Durchgangsbohrungen, bei denen unabhängig vom Endoskop 10 durch die gegenüberliegende Öffnung der Bohrung der Lichtfinger 50 eingeführt werden kann. Im Gegensatz zu handelsüblichen Lichtfingern mit quasi punktförmiger Lichtquelle an der Spitze kann auch hier durch die erfindungsgemäße Mikro-LED-Beleuchtung eine erheblich verbesserte Ausleuchtung erreicht werden. Bei Anbringung der Mikro-LEDs 30 in einer der seitlichen Abstrahlrichtungen 31.3 über einen bestimmten Bereich kann ein erheblich größerer Bereich optimal ausgeleuchtet werden.

Bei einem Aufsteckrohr werden die Mikro-LEDs 30 auf einem Rohr angebracht, das, passend zum Durchmesser des Endoskops 10, über das Endoskop 10 gesteckt wird und verschieb- und drehbar gelagert ist. Die Mikro-LEDs 30 sind am vorderen Ende des Rohres umfänglich verteilt angebracht. Im darauf folgenden Rohrabschnitt sind möglichst große Fenster 61 in die Rohrwandung eingebracht. Dies dient dazu, dass bei vorgeschobenem Rohr noch ein möglichst wenig eingeschränkter Blick auf die Wandung des Hohlraumes möglich ist. Beispielsweise können zwei Fenster 61 und zwei Stege 62 vorhanden sein, die einen vorderen Ring 63 mit den Mikro-LEDs 30 halten. An diesen Stegen 62 können auch die elektrischen Leitungen zur Versorgung der Mikro-LEDs 30 entlang geführt werden. Andere Anordnungen mit mehreren Stegen 62 sind ebenfalls möglich.

Eine weitere Möglichkeit stellt ein transparentes Aufsteckrohr, beispielsweise aus Glas oder Kunststoff, dar. Das Ausschneiden von Fenstern 61 bzw. Stegen 62 ist dann nicht erforderlich. Die elektrischen Zuleitungen 69 für die Mikro-LEDs 30 können ebenfalls in Form transparenter Leiterbahnen auf das Rohr aufgebracht werden, so dass sich umfänglich keinerlei Verdeckung durch Stege oder elektrische Leitungen ergibt. Allerdings muss die Qualität des transparenten Rohres hohen optischen Anforderungen genügen, da die Abbildung der Hohlraum-Oberflächen durch die Wand des transparenten Aufsteckrohres erfolgt. Der Durchgang der Lichtstrahlen durch die gekrümmten Oberflächen der Rohrwandung muss bei der Auslegung des distalen Objektivs 23 berücksichtigt werden.

Die Leuchtmittel können auch auf einem Lichtfinger angeordnet sein, der durch einen Instrumentenkanal des Endoskops 10 zum distalen Ende 20 vorgeschoben wird, dort aus dem Endoskop 10 austritt und so die Szene beleuchtet.

Da im beobachtbaren Bildfeld bei vorgeschobenem Beleuchtungsträger - Lichtfinger 50 oder Aufsteckrohr 60 - zwangsläufig ein gewisser Teil der Bohrungswandung verdeckt wird, kann die Bohrungswandung nicht aus einer Position vollständig rundum zu 360 Grad abgebildet werden.

Zur Abtastung der gesamten Wandungsoberfläche muss das Aufsteckrohr, das Endoskop 10 oder das Prüfteil um einen Winkel gedreht werden, so dass die zunächst durch die Stege 62 verdeckten Oberflächenbereiche in den Fenstern 61 zu liegen kommen und so ebenfalls sichtbar werden. Bei einer automatischen Bildgewinnung kann dies beispielsweise derart erfolgen, dass beim Einfahren in die Bohrung eine erste Bildsequenz erfasst wird, am Ende der Einfahr-Bewegung die besagte Drehung ausgeführt wird und beim Herausfahren aus der Bohrung eine zweite Bildsequenz aufgenommen wird, die nun die zuvor verdeckten Oberflächenbereiche enthält. Für die automatisierte Bewertung der Bohrung können nun beide Bildsequenzen getrennt ausgewertet werden und die Bewertungsergebnisse zu einer Gesamtbewertung vereinigt werden oder die beiden Bildsequenzen mit Hilfe von Registrierungsalgorithmen zunächst zusammengeführt werden, so dass sich ein ungestörtes Gesamtabbild der inneren Oberfläche ergibt, das dann einer Bildauswertung zugeführt wird.

## Patentansprüche

1. Beleuchtungsvorrichtung (19) für eine Bilderfassungseinrichtung (17) am distalen Ende (20) eines Endoskops (10), mit folgenden Merkmalen:
einem Beleuchtungsträger (50; 60), der an dem distalen Ende (20) des Endoskops (10) anordenbar ist,
einer Vielzahl von Mikro-LEDs (30), die jeweils eine Hauptoberfläche, von der die Abstrahlung erfolgt, aufweisen, die eine maximale laterale Ausdehnung von weniger als 500 µm aufweist, und die an dem Beleuchtungsträger (50; 60) so angeordnet sind, um bei elektrischer Anregung eine Umgebung des distalen Endes (20) des Endoskops (10) zumindest abschnittsweise zu beleuchten,
wobei der Beleuchtungsträger (50; 60) als ein Rohr ausgebildet ist und einen Haltebereich (65), einen Öffnungsbereich (61) und einen Beleuchtungsbereich (63) aufweist, wobei sich der Öffnungsbereich (61) seitlich zu der geometrischen Achse des Endoskops zwischen dem Beleuchtungsbereich (63) und dem Haltebereich (65) erstreckt, wobei die Mikro-LEDs (30) an dem Beleuchtungsbereich (63) angeordnet sind und der Haltebereich (65) am distalen Ende (20) des Endoskops (10) anordenbar ist, so dass ein optisches Bild der Umgebung am distalen Ende (20) des Endoskops (10) durch den seitlichen Öffnungsbereich (61) erfassbar ist.

2. Beleuchtungsvorrichtung (19) gemäß Anspruch 1, bei der die Vielzahl von Mikro-LEDs (30) als ein Array (13; 53; 63) oder zeilenförmig auf einer Außenfläche des Beleuchtungsträgers (50; 60) angeordnet sind.

3. Beleuchtungsvorrichtung (19) gemäß einem der vorhergehenden Ansprüche, bei der Beleuchtungsträger (50; 60) zumindest 10 Mikro-LEDs (30) aufweist.

4. Beleuchtungsvorrichtung (19) gemäß einem der vorhergehenden Ansprüche, bei der die Mikro-LEDs (30) jeweils eine Hauptoberfläche, von der die Abstrahlung erfolgt, aufweisen, die eine maximale laterale Ausdehnung von weniger als 300 µm, 100 µm oder 10 µm aufweist.

5. Beleuchtungsvorrichtung (19) gemäß Anspruch 4, bei der die Hauptoberfläche einer Mikro-LED (30) einen Flächeninhalt von höchsten 0,01 mm² aufweist.

6. Beleuchtungsvorrichtung (19) gemäß einem der vorhergehenden Ansprüche, bei der der Beleuchtungsträger (50; 60) mit dem distalen Ende (20) des Endoskops (10) mechanisch verbindbar ist.

7. Beleuchtungsvorrichtung (19) gemäß einem der vorhergehenden Ansprüche, bei der der Beleuchtungsträger (50; 60) an dem distalen Ende (20) des Endoskops (10) verschiebbar und/oder drehbar angeordnet ist.

8. Beleuchtungsvorrichtung (19) gemäß einem der Ansprüche 1 bis 5, bei der der Beleuchtungsträger (50; 60) durch einen Abschnitt des Endoskops (10) an dem distalen Ende (20) desselben ausgebildet ist.

9. Beleuchtungsvorrichtung (19) gemäß einem der vorhergehenden Ansprüche, bei der auf einer Oberfläche des Beleuchtungsträgers (50; 60) eine erste Gruppe von Mikro-LEDs (30.1) und eine zweite Gruppe von Mikro-LEDs (30.2) angeordnet sind, wobei die erste Gruppe von Mikro-LEDs (30.1) eine erste Hauptemissionsrichtung (31.1) aufweist und die zweite Gruppe von Mikro-LEDs (30.2) eine zweite Hauptemissionsrichtung (31.2) aufweist, wobei sich die erste und die zweite Hauptemissionsrichtung (31.1, 31.2) bezüglich der Oberfläche des Beleuchtungsträgers (50; 60) um einen Winkel α ≠ 0 unterscheidet.

10. Beleuchtungsvorrichtung (19) gemäß Anspruch 9, bei der die erste Hauptemissionsrichtung (31.1) oder die zweite Hauptemissionsrichtung (31.2) sich von einer optischen Achse (21) der Bilderfassungseinrichtung (17) an dem distalen Ende (20) des Endoskops (10) um einen Winkel γ mit 0 ≤ γ ≤ 160° unterscheidet.

11. Beleuchtungsvorrichtung (19) gemäß einem der vorhergehenden Ansprüche, bei der zumindest ein Teil der Mikro-LEDs (30) jeweils eine Mikro-Linse (33) aufweist.

12. Beleuchtungsvorrichtung (19) gemäß einem der vorhergehenden Ansprüche, bei der zumindest ein Teil der Mikro-LEDs (30) einen Diffusor (34) aufweist.

13. Beleuchtungsvorrichtung (19) gemäß einem der vorhergehenden Ansprüche, bei der der Beleuchtungsträger (50; 60) eine Vergussmasse zum Schutz der Mikro-LEDs (30) aufweist.

14. Beleuchtungsvorrichtung (19) gemäß Anspruch 13, bei der die Vergussmasse einen Diffusor (34) bildet.

15. Beleuchtungsvorrichtung (19) gemäß einem der vorhergehenden Ansprüche, bei der der Beleuchtungsträger (50; 60) transparentes Glas oder einen durchsichtigen Kunststoff aufweist.

16. Beleuchtungsvorrichtung (19) gemäß einem der vorhergehenden Ansprüche, wobei die Mikro-LEDs (30) mittels einer Trägerfolie (67) an dem Beleuchtungsträger (50; 60) angeordnet sind.

17. Beleuchtungsvorrichtung (19) gemäß Anspruch 16, wobei die Trägerfolie Leiterbahnen (67) zur Kontaktierung der Mikro-LEDs (30) aufweist.

18. Beleuchtungsvorrichtung (19) gemäß Anspruch 17, bei der die Leiterbahnen transparent ausgebildet sind.

19. Beleuchtungsvorrichtung (19) gemäß einem der vorhergehenden Ansprüche, bei der zumindest ein Teil der Mikro-LEDs (30) gepulst ansteuerbar sind.

20. Beleuchtungsvorrichtung (19) gemäß einem der vorhergehenden Ansprüche, bei der zumindest ein Teil der Mikro-LEDs (30) voneinander getrennt ansteuerbar sind.

21. Beleuchtungsvorrichtung (19) gemäß einem der vorhergehenden Ansprüche, bei der unterschiedliche Mikro-LEDs (30) Licht mit unterschiedlichen Wellenlängen emittieren.

22. Beleuchtungsvorrichtung (19) gemäß einem der vorhergehenden Ansprüche, bei der die Bilderfassungseinrichtung (17) eine Optik mit einem Objektiv (23) am distalen Ende (20) aufweist.

23. Beleuchtungsvorrichtung (19) gemäß Anspruch 22, bei der die Bilderfassungseinrichtung (17) eine Optik mit einen Rundum-Retroblick (25) am distalen Ende (20) für eine Rundumerfassung aufweist.

24. Beleuchtungsvorrichtung (19) gemäß einem der vorhergehenden Ansprüche, bei der die Bilderfassungseinrichtung (17) eine CCD-Kamera (27) aufweist.

25. Beleuchtungsvorrichtung (19) gemäß einem der vorhergehenden Ansprüche, wobei der Beleuchtungsträger (50; 60) an dem distalen Ende (20) des Endoskops (10) entlang oder parallel zu der geometrischen Achse in unterschiedliche Positionen relativ zu dem distalen Ende (20) des Endoskops (10) verschiebbar ist.

26. Verfahren zum Betreiben einer Beleuchtungsvorrichtung (19) für eine Bilderfassungseinrichtung (17) gemäß einem der vorhergehenden Ansprüche, mit folgenden Schritten:
Beleuchten zumindest eines Abschnittes einer Umgebung des distalen Endes (20) des Endoskops (10) durch eine elektrischen Anregung der Mikro-LEDs (30).

27. Verfahren gemäß Anspruch 26, mit folgenden Schritten:
gepulstes Ansteuern zumindest eines Teils der Mikro-LEDs (30)

28. Verfahren gemäß Anspruch 26 oder Anspruch 27, mit folgenden Schritten:
getrenntes Ansteuern zumindest eines Teils der Mikro-LEDs (30)

29. Verfahren gemäß einem der Ansprüche 26 bis 28, ferner mit folgendem Schritt:
Verschieben des Beleuchtungsträgers, um eine Veränderung einer Ausleuchtung der Umgebung des distalen Endes zu erreichen.

30. Verfahren zur Herstellung einer Beleuchtungsvorrichtung (19) für eine Bilderfassungseinrichtung (17) am distalen Ende (20) eines Endoskops (10), mit folgenden Schritten:
Bereitstellen eines Arrays von Mikro-LEDs (30) auf einer Trägerfolie (67), wobei die Mikro-LEDs jeweils eine Hauptoberfläche, von der die Abstrahlung erfolgt, aufweisen, die eine maximale laterale Ausdehnung von weniger als 500 µm aufweist; und
Anordnen der Trägerfolie (67) auf einen Beleuchtungsträger (50; 60), der an dem distalen Ende (20) des Endoskops (10) anordenbar ist, um bei elektrischer Anregung der Mikro-LEDs eine Umgebung des distalen Endes (20) des Endoskops (10) zumindest abschnittsweise zu beleuchten, wobei der Beleuchtungsträger (50; 60) als ein Rohr ausgebildet ist und einen Haltebereich (65), einen Öffnungsbereich (61) und einen Beleuchtungsbereich (63) aufweist, wobei sich der Öffnungsbereich (61) seitlich zu der geometrischen Achse des Endoskops zwischen dem Beleuchtungsbereich (63) und dem Haltebereich (65) erstreckt, wobei die Mikro-LEDs (30) an dem Beleuchtungsbereich (63) angeordnet sind und der Haltebereich (65) am distalen Ende (20) des Endoskops (10) anordenbar ist, so dass ein optisches Bild der Umgebung am distalen Ende (20) des Endoskops (10) durch den seitlichen Öffnungsbereich (61) erfassbar ist.

31. Verfahren nach Anspruch 30, wobei die Trägerfolie so angeordnet wird, dass das Array von Mikro-LEDs (30) eine erste und zweite benachbarte Gruppe von Mikro-LEDs (30) auf einem Oberflächenbereichs des Beleuchtungsträgers (50; 60) mit einer ersten und zweiten Hauptabstrahlrichtung (31.1, 31.2), die bezüglich des Oberflächenbereichs des Beleuchtungsträgers unterschiedlich ist, aufweist.

## Claims

1. Illumination apparatus (19) for an image sensor (17) at a distal end (20) of an endoscope (10), comprising:
an illumination carrier (50; 60) which is arrangeable at the distal end (20) of the endoscope (10),
a multitude of micro LEDs (30) each comprising a main surface comprising a maximum lateral extent of less than 500 µm from which the emission is effected, and which are arranged on the illumination carrier (50; 60) such as to illuminate, with electrical excitation, at least some portions of an environment of the distal end (20) of the endoscope (10),
wherein the illumination carrier (50; 60) is formed as a tube and comprises a support region (65), an opening region (61), and an illumination region (63), the opening region (61) laterally extending to the geometrical axis of the endoscope between the illumination region (63) and the support region (65), wherein the micro LEDs (30) are arranged at the illumination region (63) and the support region (65) is arrangeable at the distal end (20) of the endoscope (10), so that an optical image of the environment at the distal end (20) of the endoscope (10) may be sensed through the lateral opening region (61).

2. Illumination apparatus (19) according to claim 1, wherein the multitude of micro LEDs (30) are arranged as an array (13; 53; 63) or in rows on an outer surface of the illumination carrier (50; 60).

3. Illumination apparatus (19) according to any one of the preceding claims, wherein the illumination carrier (50; 60) comprises at least 10 micro LEDs (30).

4. Illumination apparatus (19) according to any one of the preceding claims, wherein the micro LEDs (30) each comprise a main surface comprising a maximum lateral extent of less than 300 µm, 100 µm, or 10 µm from which the emission is effected.

5. Illumination apparatus (19) according to claim 4, wherein the main surface of a micro LED (30) comprises an area of 0.01 mm² at the most.

6. Illumination apparatus (19) according to any one of the preceding claims, wherein the illumination carrier (50; 60) is mechanically connectable to the distal end (20) of the endoscope (10).

7. Illumination apparatus (19) according to any one of the preceding claims, wherein the illumination carrier (50; 60) is shiftably and/or rotatably arranged at the distal end (20) of the endoscope (10).

8. Illumination apparatus (19) according to any one of claims 1 to 5, wherein the illumination carrier (50; 60) is formed by a portion of the endoscope (10) at the distal end (20) thereof.

9. Illumination apparatus (19) according to any one of the preceding claims, wherein a first group of micro LEDs (30.1) and a second group of micro LEDs (30.2) are arranged on a surface of the illumination carrier (50; 60), wherein the first group of micro LEDs (30.1) comprises a first main emission direction (31.1) and the second group of micro LEDs (30.2) comprises a second main emission direction (31.2), wherein the first and the second main emission directions (31.1, 31.2) differ with respect to the surface of the illumination carrier (50; 60) by an angle α ≠ 0°.

10. Illumination apparatus (19) according to claim 9, wherein the first main emission direction (31.1) or the second main emission direction (31.2) differ from an optical axis (21) of the image sensor (17) at the distal end (20) of the endoscope (10) by an angle γ with 0° ≤ γ ≤ 160°.

11. Illumination apparatus (19) according to any one of the preceding claims, wherein at least part of the micro LEDs (30) comprise a micro lens (33).

12. Illumination apparatus (19) according to any one of the preceding claims, wherein at least part of the micro LEDs (30) comprise a diffuser (34).

13. Illumination apparatus (19) according to any one of the preceding claims, wherein the illumination carrier (50; 60) comprises a compound for protecting the micro LEDs (30).

14. Illumination apparatus (19) according to claim 13, wherein the compound forms a diffuser (34).

15. Illumination apparatus (19) according to any one of the preceding claims, wherein the illumination carrier (50; 60) comprises transparent glass or translucent plastic.

16. Illumination apparatus (19) according to any one of the preceding claims, wherein the micro LEDs (30) are arranged on the illumination carrier (50; 60) by means of a carrier foil (67).

17. Illumination apparatus (19) according to claim 16, wherein the carrier foil comprises conductor traces (67) for contacting the micro LEDs (30).

18. Illumination apparatus (19) according to claim 17, wherein the conductor traces are formed transparently.

19. Illumination apparatus (19) according to any one of the preceding claims, wherein at least part of the micro LEDs (30) are controllable in a pulsed manner.

20. Illumination apparatus (19) according to any one of the preceding claims, wherein at least part of the micro LEDs (30) are controllable separately from each other.

21. Illumination apparatus (19) according to any one of the preceding claims, wherein different micro LEDs (30) emit light at different wavelengths.

22. Illumination apparatus (19) according to any one of the preceding claims, wherein the image sensor (17) comprises optics with an objective (23) at the distal end (20).

23. Illumination apparatus (19) according to claim 22, wherein the image sensor (17) comprises optics with an all-round backward look (25) at the distal end (20) for an all-round sensing.

24. Illumination apparatus (19) according to any one of the preceding claims, wherein the image sensor (17) comprises a CCD camera (27).

25. Illumination apparatus (19) according to any one of the preceding claims, wherein the illumination carrier (50; 60) at the distal end (20) of the endoscope (10) is shiftable along or parallel to the geometrical axis to different positions with respect to the distal end (20) of the endoscope (10).

26. Method for operating an illumination apparatus (19) for an image sensor (17) according to any one of the preceding claims, comprising:
illuminating, by electrical excitation of the micro LEDs (30), at least a portion of an environment of the distal end (20) of the endoscope (10).

27. Method according to claim 26, comprising:
controlling, in a pulsed manner, at least part of the micro LEDs (30).

28. Method according to claim 26 or claim 27, comprising:
separately controlling at least part of the micro LEDs (30).

29. Method according to any one of claims 26 to 28, further comprising:
shifting the illumination carrier in order to obtain a change in illumination of the environment of the distal end.

30. Method for manufacturing an illumination apparatus (19) for an image sensor (17) at a distal end (20) of an endoscope (10), comprising:
providing an array of micro LEDs (30) on a carrier foil (67), wherein the micro LEDs each comprise a main surface comprising a maximum lateral extent of less than 500 µm from which the emission is effected; and
arranging the carrier foil (67) on an illumination carrier (50; 60) which is arrangeable at the distal end (20) of the endoscope (10), such as to illuminate, with electrical excitation of the micro LEDs, at least some portions of an environment of the distal end (20) of the endoscope (10), wherein the illumination carrier (50; 60) is formed as a tube and comprises a support region (65), an opening region (61), and an illumination region (63), the opening region (61) laterally extending to the geometrical axis of the endoscope between the illumination region (63) and the support region (65), wherein the micro LEDs (30) are arranged at the illumination region (63) and the support region (65) is arrangeable at the distal end (20) of the endoscope (10), so that an optical image of the environment at the distal end (20) of the endoscope (10) may be sensed through the lateral opening region (61).

31. Method according to claim 30, wherein the carrier foil is arranged such that the array of micro LEDs (30) comprises a first and a second adjacent group of micro LEDs (30) on a surface region of the illumination carrier (50; 60), having a first and a second main emission direction (31.1, 31.2), which differ with respect to the surface region of the illumination carrier.

## Revendications

1. Dispositif d'éclairage (19) pour un dispositif de capture d'image (17) à l'extrémité distale (20) d'un endoscope (10), aux caractéristiques suivantes:
un support d'éclairage (50; 60) qui peut être disposé à l'extrémité distale (20) de l'endoscope (10),
une pluralité de micro-LED (30) présentant, chacune, une surface principale à partir de laquelle a lieu le rayonnement, laquelle présente une extension latérale maximale de moins de 500 µm, et qui sont disposées sur le support d'éclairage (50; 60) de manière à éclairer, en cas d'excitation électrique, au moins par segments un environnement de l'extrémité distale (20) de l'endoscope (10),
dans lequel le support d'éclairage (50; 60) est réalisé sous forme d'un tube 20 et présente une zone de maintien (65), une zone d'ouverture (61) et une zone d'éclairage (63), la zone d'ouverture (61) s'étendant latéralement par rapport à l'axe géométrique de l'endoscope, entre la zone d'éclairage (63) et la zone de maintien (65), les micro-LED (30) étant disposées sur la région d'éclairage (63) et la zone de maintien (65) pouvant être disposée à l'extrémité distale (20) de l'endoscope (10) de sorte qu'une image optique de l'environnement à l'extrémité distale (20) de l'endoscope (10) puisse être captée à travers la zone d'ouverture latérale (61).

2. Dispositif d'éclairage (19) selon la revendication 1, dans lequel la pluralité de micro-LED (30) sont disposées en forme de réseau (13; 53; 63) ou en forme de lignes sur une surface extérieure du support d'éclairage (50; 60).

3. Dispositif d'éclairage (19) selon l'une des revendications précédentes, dans lequel le support d'éclairage (50; 60) présente au moins 10 micro-LED (30).

4. Dispositif d'éclairage (19) selon l'une des revendications précédentes, dans lequel les micro-LED (30) présentent, chacune, une surface principale à partir de laquelle a lieu le rayonnement, laquelle présente une extension latérale maximale de moins de 300 µm, de 100 µm ou de 10 µm.

5. Dispositif d'éclairage (19) selon la revendication 4, dans lequel la surface principale d'une micro-LED (30) présente une aire surfacique de tout au plus 0,01 mm².

6. Dispositif d'éclairage (19) selon l'une des revendications précédentes, dans lequel le support d'éclairage (50; 60) peut être connecté mécaniquement à l'extrémité distale (20) de l'endoscope (10).

7. Dispositif d'éclairage (19) selon l'une des revendications précédentes, dans lequel le support d'éclairage (50; 60) est disposé de manière déplaçable et/ou rotative à l'extrémité distale (20) de l'endoscope (10).

8. Dispositif d'éclairage (19) selon l'une des revendications 1 à 5, dans lequel le support d'éclairage (50; 60) est réalisé par un segment de l'endoscope (10) à l'extrémité distale (20) de ce dernier.

9. Dispositif d'éclairage (19) selon l'une des revendications précédentes, dans lequel sont disposés, sur une surface du support d'éclairage (50, 60), un premier groupe de micro-LED (30.1) et un deuxième groupe de micro-LED (30.2), le premier groupe de micro-LED (30.1) présentant une première direction d'émission principale (31.1) et le deuxième groupe de micro-LED (30.2) présentant une deuxième direction d'émission principale (31.2), la première et la deuxième direction d'émission principale (31.1, 31.2) différant par rapport à la surface du support d'éclairage (50; 60) d'un angle *α* ≠ 0.

10. Dispositif d'éclairage (19) selon la revendication 9, dans lequel la première direction d'émission principale (31.1) ou la deuxième direction d'émission principale (31.2) différant d'un axe optique (21) du dispositif de capture d'image (17) à l'extrémité distale (20) de l'endoscope (10) d'un angle *γ* avec 0 ≤ *γ* ≤ 160°.

11. Dispositif d'éclairage (19) selon l'une des revendications précédentes, dans lequel au moins une partie des micro-LED (30) présentent, chacune, une microlentille (33).

12. Dispositif d'éclairage (19) selon l'une des revendications précédentes, dans lequel au moins une partie des micro-LED (30) présentent un diffuseur (34).

13. Dispositif d'éclairage (19) selon l'une des revendications précédentes, dans lequel le support d'éclairage (50; 60) présente une masse de scellement pour protéger les micro-LED (30).

14. Dispositif d'éclairage (19) selon la revendication 13, dans lequel la masse de scellement constitue un diffuseur (34).

15. Dispositif d'éclairage (19) selon l'une des revendications précédentes, dans lequel le support d'éclairage (50; 60) présente du verre transparent ou une matière plastique transparente.

16. Dispositif d'éclairage (19) selon l'une des revendications précédentes, dans lequel les micro-LED (30) sont disposées au moyen d'un film de support (67) sur le support d'éclairage (50; 60).

17. Dispositif d'éclairage (19) selon la revendication 16, dans lequel le film de support (67) présente des chemins de conducteurs pour la mise en contact des micro-LED (30).

18. Dispositif d'éclairage (19) selon la revendication 17, dans lequel les chemins de conducteurs sont réalisés transparents.

19. Dispositif d'éclairage (19) selon l'une des revendications précédentes, dans lequel au moins une partie des micro-LED (30) peuvent être activées de manière pulsée.

20. Dispositif d'éclairage (19) selon l'une des revendications précédentes, dans lequel au moins une partie des micro-LED (30) peuvent être activées séparément l'une de l'autre.

21. Dispositif d'éclairage (19) selon l'une des revendications précédentes, dans lequel différentes micro-LED (30) émettent de la lumière de longueurs d'onde différentes.

22. Dispositif d'éclairage (19) selon l'une des revendications précédentes, dans lequel le moyen de capture d'image (17) présente une optique avec un objectif (23) à l'extrémité distale (20).

23. Dispositif d'éclairage (19) selon la revendication 22, dans lequel le dispositif de capture d'image (17) présente une optique avec un regard en arrière panoramique (25) à l'extrémité distale (20) pour une capture panoramique.

24. Dispositif d'éclairage (19) selon l'une des revendications précédentes, dans lequel le moyen de capture d'image (17) présente une caméra CCD (27).

25. Dispositif d'éclairage (19) selon l'une des revendications précédentes, dans lequel le support d'éclairage (50; 60) peut être déplacé à l'extrémité distale (20) de l'endoscope (10) le long de ou parallèlement à l'axe géométrique dans différentes positions par rapport à l'extrémité distale (20) de l'endoscope (10).

26. Procédé permettant de faire fonctionner un dispositif d'éclairage (19) pour un dispositif de capture d'image (17) selon l'une des revendications précédentes, aux étapes suivantes consistant à:
éclairer au moins un segment d'un environnement de l'extrémité distale (20) de l'endoscope (10) par une excitation électrique des micro-LED (30).

27. Procédé selon la revendication 26, aux étapes suivantes consistant à:
activer de manière pulsée au moins une partie des micro-LED (30).

28. Procédé selon la revendication 26 ou la revendication 27, aux étapes suivantes consistant à:
activer séparément au moins une partie des micro-LED (30).

29. Procédé selon l'une quelconque des revendications 26 à 28, par ailleurs à l'étape suivante consistant à:
déplacer le support d'éclairage pour obtenir une modification d'un éclairage de l'environnement de l'extrémité distale.

30. Procédé de fabrication d'un dispositif d'éclairage (19) pour un dispositif de capture d'image (17) à l'extrémité distale (20) d'un endoscope (10), aux étapes suivantes consistant à:
prévoir un réseau de micro-LED (30) sur un film de support (67), les micro-LED présentant, chacune, une surface principale à partir de laquelle a lieu le rayonnement, laquelle présente une extension latérale maximale de moins de 500 µm; et
disposer le film de support (67) sur un support d'éclairage (50; 60) qui peut être disposé à l'extrémité distale (20) de l'endoscope (10), pour éclairer, en cas d'excitation électrique des micro-LED, au moins par segments un environnement de l'extrémité distale (20) de l'endoscope (10), le support d'éclairage (50; 60) étant réalisé sous forme d'un tube et présentant une zone de maintien (65), une zone d'ouverture (61) et une zone d'éclairage (63), la zone d'ouverture (61) s'étendant latéralement par rapport à la l'axe géométrique de l'endoscope, entre la zone d'éclairage (63) et la zone de maintien (65), les micro-LED (30) étant disposées dans la zone d'éclairage (63) et la zone de maintien (65) pouvant être disposée à l'extrémité distale (20) de l'endoscope (10) de sorte qu'une image optique de l'environnement à l'extrémité distale (20) de l'endoscope (10) puisse être captée à travers la zone d'ouverture latérale (61).

31. Procédé selon la revendication 30, dans lequel le film de support est disposé de sorte que le réseau de micro-LED (30) présente un premier et un deuxième groupe adjacent de micro-LED (30) sur une zone de surface du support d'éclairage (50; 60) avec une première et une deuxième direction d'émission principale (31.1, 31.2) différentes par rapport à la surface du support d'éclairage.
